# EUROPEAN PATENT APPLICATION

(11) **EP 2 665 009 A1**
(43) Date of publication of application: **20.11.2013**
(21) Application number: 11855596.0
(22) Date of filing: 27.06.2011
(51) Int. Cl.: G06F 19/20, G01N 33/53, G06F 17/30

(54) **NUCLEIC ACID INFORMATION PROCESSING DEVICE AND PROCESSING METHOD THEREOF**

(30) Priority: 11.01.2011 JP 2011003106
(71) Applicant: Japan Software Management Co., Ltd., Yokohama-shi Kanagawa 221-0056 (JP); Bioinformatics Institute for Global Good Inc., Tokyo 140-0001 (JP)
(72) Inventor: NASU, Hisanori, Yokohama-shi Kanagawa 221-0056 (JP); TSUJIMOTO, Atsumi, Yokohama-shi Kanagawa 221-0056 (JP); YAMAKAWA, Takehiro, Yokohama-shi Kanagawa 221-0056 (JP); ONO, Hiroaki, Yokohama-shi Kanagawa 221-0056 (JP)
(74) Representative: Williams, Aylsa
(86) International application number: PCT/JP2011/064685
(87) International publication number: WO 2012/096016

(57) **Abstract**

It is an object to easily obtain hybridization results using a probe set with no time limit for use, that is equivalent to a DNA microarray. A nucleic acid information processing device includes: a storage unit that stores first base sequence information including information on a plurality of base sequences, and second base sequence information including information on a plurality of base sequences; a threshold value receiving unit adapted to receive information that identifies a similarity threshold; a hybridization unit adapted to identify a degree of similarity and a starting position and a finishing position of a similar portion for one to one combinations with the base sequences included in the first base sequence information as a target, and the base sequences included in the second base sequence information as a probe; and a similar base sequence counting unit adapted to count for each probe a number of the targets for which the identified degree of similarity is greater than or equal to the similarity threshold, and storing the count in the storage unit.

## Description

### BACKGROUND OF THE INVENTION

The present invention relates to technology for processing nucleic acid information. The present invention claims priority from Japanese Patent Application Number 2011-3106 filed on January 11, 2011, and the content of that application is hereby incorporated by reference into the present application, for designated countries that recognize incorporation of documents by reference.

There are a huge number of genes and a huge number of types of genes within biosystems such as biological populations, individuals, body tissues, and cells, and whose existence is maintained while their products affect each other. Conventionally, analysis of the presence or variation of individual genes is executed for individual genes using test methods in which a single gene is investigated with a single test, as represented by southern blotting and northern blotting. However, with the prevalence of deoxyribonucleic acid (DNA) microarrays (in this application, considered to be synonymous with DNA chip, for convenience), it became possible to comprehensively deal with the presence and expression level of much genetic information in one physical and physiological test. On the other hand, with the progress of the genome project which preceded this, in the technology for determining DNA base sequences, a family of apparatus known as next generation sequencers was commercialized that enormously increased the numbers of DNA fragments that could be analyzed in parallel at the same time. As a result of this family of apparatus, the numbers of DNA fragments and bases that could be analyzed by a single operation of a next generation sequencer increased dramatically. Such technology is disclosed in Patent Document 1.

### PRIOR ART DOCUMENTS

### Patent Document

Patent Document 1: Japanese Unexamined Patent Application Publication No. 2010-193832A

### SUMMARY OF THE INVENTION

### Problems to be Solved by the Invention

However, the DNA microarray as described above is an extremely useful experimental tool, but it is considered that it has three problems. The first is that frequency analysis results of similar sequences using a DNA microarray do not have 100% reproducibility, so it cannot be said to have high accuracy. The second is that in tests using a DNA microarray, the quantity of hybridized target molecules with the probe molecules can be measured, but it is not possible to obtain target molecule base sequence information. There is various detailed information that cannot be obtained from a hybridization test using a DNA microarray only, such as what part of the target molecule base sequence has been hybridized with each probe base sequence, was the match of the base sequence of the hybridized part with the probe molecule base sequence 100%, were there mismatches, and if there were mismatches, where were they. The third is that DNA microarrays and target nucleic acid used in DNA microarray tests cannot be reused under the same conditions.

With the foregoing conventional technology in view, it is an object of the present invention to easily obtain hybridization results using a probe set with no time limit for use, that is equivalent to a DNA microarray.

For example, a nucleic acid information processing device according to the present invention includes: a storage unit that stores first base sequence information that includes information of a plurality of base sequences, and second base sequence information that includes information of a plurality of base sequences; a threshold value receiving unit adapted to receive information that identifies a similarity threshold; a hybridization unit adapted to identify the degree of similarity and a starting position and a finishing position of a similar portion for one to one combinations with the base sequences included in the first base sequence information as a target, and the base sequences included in the second base sequence information as a probe; and a similar base sequence counting unit adapted to count for each probe a number of the targets for which the identified degree of similarity is greater than or equal to the similarity threshold, and storing the count in the storage unit.

Also, for example, in a method of processing nucleic acid information with a nucleic acid information processing device, the nucleic acid information processing device includes: a storage unit that stores first base sequence information that includes information of a plurality of base sequences, and second base sequence information that includes information of a plurality of base sequences, and a processing unit, the processing unit executes: a threshold value receiving step of receiving information that identifies a similarity threshold; a hybridization step of identifying the degree of similarity and a starting position and a finishing position of a similar portion for one to one combinations with the base sequences included in the first base sequence information as a target, and the base sequences included in the second base sequence information as a probe; and a similar base sequence counting step of counting for each probe a number of the targets for which the identified degree of similarity is greater than or equal to the similarity threshold, and storing the count in the storage unit.

By applying the present invention, it is possible to easily obtain hybridization results using a probe set with no time limit for use, that is equivalent to a DNA microarray.

### BRIEF DESCRIPTION OF THE DRAWINGS

FIG. 1 is a schematic view illustrating a method of processing nucleic acid information according to this embodiment.
FIG. 2 is a schematic view illustrating a hybridization process of the method of processing nucleic acid information according to this embodiment.
FIG. 3 is a schematic view illustrating the hybridization process according to this embodiment.
FIG. 4 is a schematic view illustrating a virtual hybridization process of the method of processing nucleic acid information according to this embodiment.
FIG. 5 is a functional block diagram of a nucleic acid information processing device according to this embodiment.
FIG. 6 is a view illustrating a data structure of a target fragment storage unit.
FIG. 7 is a view illustrating a data structure of a probe storage unit.
FIG. 8 is a view illustrating a data structure of a degree of similarity storage unit.
FIG. 9 is a view illustrating a data structure of a hybridization results storage unit.
FIG. 10 is a view illustrating a data structure of a cluster storage unit.
FIG. 11 is a view illustrating a hardware configuration of the nucleic acid information processing device according to this embodiment.
FIG. 12 is a view illustrating a process flow of a clustering process.
FIG. 13 is a view illustrating a process flow of the clustering process.
FIG. 14 is a view illustrating a process flow of a virtual hybridization process.
FIG. 15 is a view illustrating a process flow of a complete hybrid identification process.
FIG. 16 is a view illustrating a process flow of a target comparison process.
FIG. 17 is a view illustrating an example of a clustering process screen.
FIG. 18 is a view illustrating an example of a clustering process results screen.
FIG. 19 is a view illustrating an example of the clustering process results screen.
FIG. 20 is a view illustrating an example of the clustering process results screen.
FIG. 21 is a view illustrating an example of a virtual hybridization process results screen.
FIG. 22 is a view illustrating an example of the virtual hybridization process results screen.
FIG. 23 is a schematic view illustrating a target comparison process.
FIG. 24 is a view illustrating an example of a process results screen of the target comparison process.
FIG. 25 is a view illustrating an example of the process results screen of the target comparison process.
FIG. 26 is a view illustrating a target counting method in a virtual hybridization process.

### DETAILED DESCRIPTION OF THE INVENTION

The cause of the first problem in the technology as described above is considered to be an accumulation of errors in the number of probe molecules and probe sequences fixed to a substrate or a matrix for each probe, each array, and each prepared lot, and errors in the physicochemical conditions for each hybridization, and the like. It is considered that the errors in the number of molecules fixed for each probe and each array are caused by differences in the equipment and enzymes for fixing and the chemical reaction fixing efficiency for each probe and each array when fixing the probe DNA to the substrate or the matrix, so that, as a result, the number of spot molecules differs for each spot between probes and between arrays.

Also, it is considered that the errors for each hybridization are caused by differences in each of the conditions for each hybridization, as it is difficult to strictly reproduce all the physicochemical conditions for each hybridization, such as the temperature, pH, ion strength, formamide concentration, probe strand length, probe quantity, target DNA concentration, whether the probe and/or the target nucleic acid is double strand or single strand, and the like, in the hybridization and the subsequent DNA microarray washing.

Always having all these conditions strictly the same in every test is extremely difficult, so it is considered that the reproducibility of frequency analysis results for similar base sequences using a DNA microarray is not 100%. Actually, when analysis is executed using this technology, it is necessary to execute the analysis using approximate values, such as the average values from the results of testing a plurality of times.

In a first embodiment of the invention according to the present application as described below, hybridization is executed virtually, in other words, it is executed as a process on a computer, using electronic information of base sequences. Therefore, in the hybridization as described above, physicochemical conditions do not play a role, so the errors and the like resulting from the conditions do not arise. Therefore, the first problem can be solved.

The cause of the second problem in the technology as described above is that in DNA microarray tests, the quantity of hybridized target nucleic acid with the probe can be measured, but it is not possible to obtain base sequence information for the target nucleic acid.

Therefore, time and effort is required to execute further analysis, to pick up the probe sequence obtained in the test results of interest, and based on this, to recover new nucleic acid having a base sequence with a high degree of similarity to the probe base sequence from the target and to determine the base sequence.

In the embodiment of the invention according to the present application as described below, hybridization is executed virtually using electronic information of base sequences. Therefore, the detailed results of the hybridization as described above are clear, and there are no uncertainties. Therefore, the second problem can be solved.

Regarding the above third technical problem, the exact same target does not exist, so it is not possible to obtain the same target again, and there is a limit to the number of DNA microarrays prepared at one time, so after these have been used up, it is necessary to prepare again another lot of DNA microarrays. This operation requires effort and cost, and, at the same time, errors between prepared lots occur, which causes the first problem as described above.

In an embodiment of the invention according to the present application as described below, hybridization is executed virtually, in other words, it is executed as a process on a computer, using electronic information of base sequences, so preservation of the target itself is not considered. Also, replication and reproduction of the base sequence of the same target is comparatively easy. Therefore, the third problem can be solved.

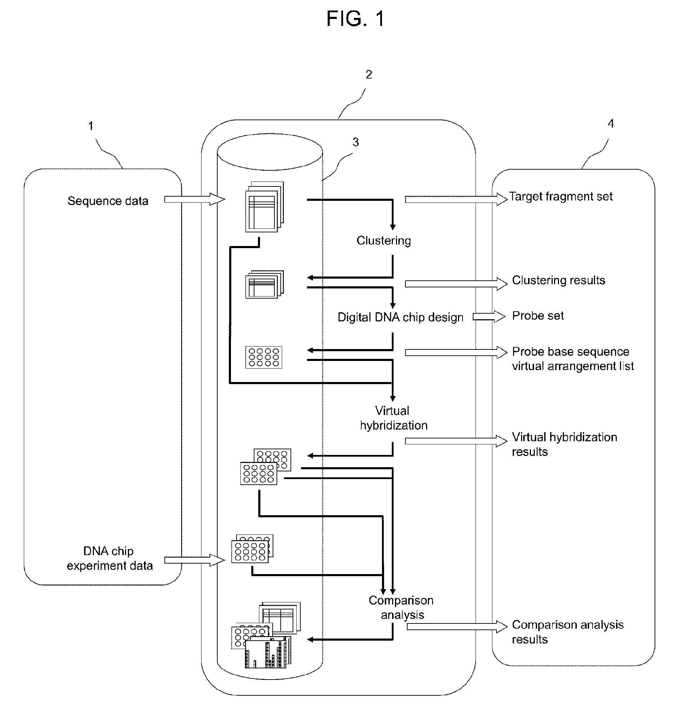

The following is a description of a first embodiment according to the present invention using FIGS. 1 to 25.

FIG. 1 is a schematic view illustrating nucleic acid information processing using a nucleic acid information processing device 100, which is an example of the first embodiment of the present invention. Specifically, FIG. 1 is a diagram illustrating a flow of frequency analysis of similar base sequences and a comparison of nucleic acid information in a digital DNA chip (DNA microarray using digital data).

Sequence data which is target fragment base sequence information output from a sequencer and DNA chip experiment data obtained in tests using a DNA chip are imported into import data 1. A processing function 2 of the nucleic acid information processing device 100 executes processing using a database 3 in which the imported sequence data and DNA chip experiment data as well as the various analysis results as described below that were executed using these data is stored.

The processing function 2 includes a function for executing a clustering process on the sequence data; a digital DNA chip design function for designing a digital DNA chip including preparing a probe base sequence list based on the clustered data and arranging it on a virtual plane; a virtual hybridization function that receives the target fragment base sequence information output from the sequencer, and analyzes the degree of similarity and frequency of the probe base sequence list; and a function for comparing the frequency analysis results for a plurality of similar base sequences, including any combination of virtual hybridization results with virtual hybridization results, imported DNA chip experiment data with imported DNA chip experiment data, or virtual hybridization results with DNA chip experiment data, in accordance with the analysis flow.

Also, the processing function 2 includes a function for outputting various analysis results for the above functions and displaying them on a computer screen. The data output includes target fragment sets, clustering results, probe sets, probe base sequence virtual arrangement lists, virtual hybridization results, comparison analysis results, and the like, as indicated by output data 4.

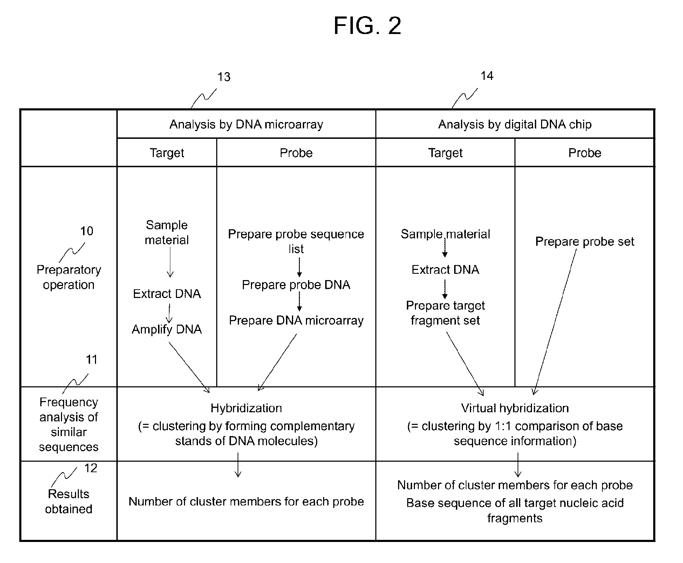

FIG. 2 is a schematic view illustrating a hybridization process of the method of processing nucleic acid information. Specifically, in FIG. 2, preparatory operations 10, frequency analysis of similar base sequences 11, and the obtained results 12 are arranged for analysis by DNA microarray 13 and analysis by digital DNA chip 14.

In analysis by DNA microarray, material sampling, DNA extraction, and DNA amplification are executed as target preparatory operations 10. Also, preparation of probe sequence list, preparation of probe DNA, and preparation of DNA microarray are executed as probe preparatory operations. Then, in the frequency analysis of similar base sequences 11, so-called hybridization of target DNA and a DNA microarray is executed.

This hybridization uses the property that a complementary strand is formed by hydrogen bonding of the base sequence of a single strand provided by a DNA microarray and the base sequence of a single strand of a complementary target. This is not limited to a complementary strand, but a positive reaction can also be obtained for a single strand of a target having the same base sequence as the base sequence provided by the DNA microarray. The obtained results 12 include the number of cluster members for each probe.

In the analysis by digital DNA chip 14, material sampling, DNA extraction, and preparation of target fragment sets are executed as target preparatory operations 10. The target fragments are identified by identifying the sequence data of bases by a sequencer, for base sequences. Also, probe sets are prepared as a probe preparatory operation. For preparing probe sets, data for target fragment sets prepared in the past may be reconfigured, or data from an existing genome database, for example, public databases such as the data of the various databases of the Genomics & Genetics at the Sanger Institute (http://www.sanger.ac.uk/genetics/), and the data of the Visualization and Analysis of Microbial Population Structures (VAMPS) database (http://vamps.mbl.edu/), or each research institute's own database that is not open to the public, and the like, may be used. In the frequency analysis of similar base sequences 11, virtual hybridization is executed in which the target fragment base sequence data and the probe set base sequence data are compared one to one.

In the virtual hybridization, using the complementarity of the bases, for each target fragment base sequence, a matching process is executed based on similarity of complementary base sequence of the probe set and the non-complementary base sequence of the probe set, to identify the corresponding combinations. The obtained results 12 include the number of cluster members for each probe, and base sequence information for all nucleic acid fragments of the target. Also, the base sequence information used as the probe set is not lost, but can be used again.

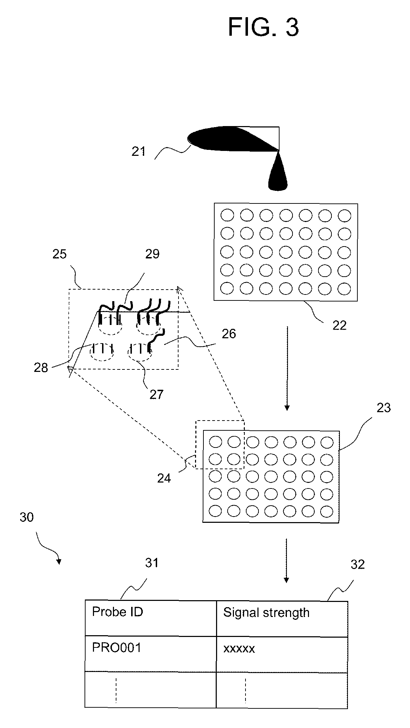

FIG. 3 is a schematic view illustrating a hybridization process in a flow of frequency analysis of a degree of similarity using the DNA microarray.

Normally, in the hybridization process, a hybridization test is executed based on the extent of complementarity between the nucleic acid molecules of each probe and target, using a labelled target nucleic acid solution 21 and a DNA microarray 22. In this case, in the hybridization test using the DNA microarray, in the hybridization and the subsequent DNA microarray washing step, the threshold for complementarity is determined depending on the physicochemical conditions (temperature, pH, ion strength, formamide concentration, probe strand length, probe quantity, target nucleic acid concentration, whether the nucleic acid of the probe and/or the target is a single strand or double strand, and the like) of each test unit.

When the hybridization test is executed, a reaction result such as, for example, a hybridized DNA microarray 23 is obtained. If a portion 24 of the DNA microarray is enlarged, as indicated in an enlarged view 25 of the hybridization results of the portion of the DNA microarray, probe DNA fragments 28 are fixed to a probe spot area 27 of a substrate 26 of the DNA microarray. Also, when the complementarity of the probe DNA fragment and the target nucleic acid fragment is greater than the threshold value of complementarity determined by the physicochemical conditions as described above, the probe DNA fragment and the target nucleic acid fragment form a double strand. As a result of this reaction, the physicochemical result that a label signal for each spot varies in strength in accordance with the number of molecules of hybridized labeled target nucleic acid fragments 29 is obtained.

In the hybridization using the DNA microarray, normally, after hybridization ranging from several hours to overnight, the washing operation is executed, so almost one day is required. In the analysis using the DNA microarray, information 30 on the approximate number (information represented by signal strength 32) of target fragments that formed double strands for each probe 31 is obtained.

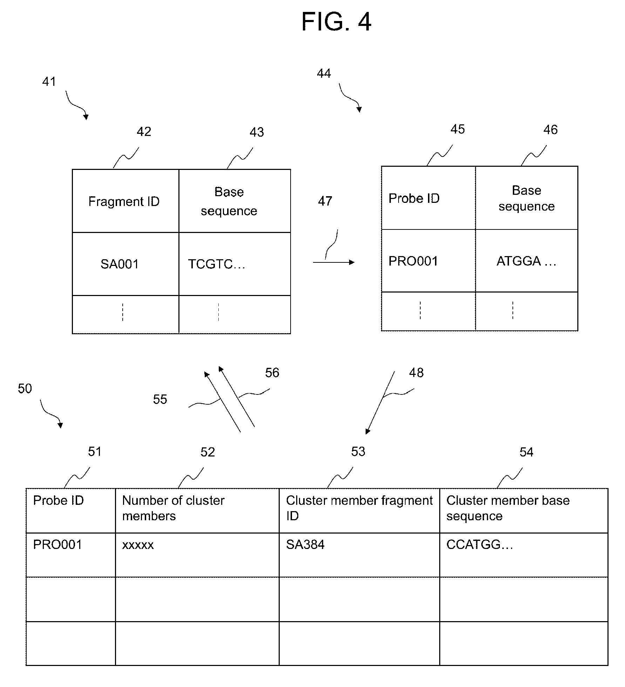

FIG. 4 is a schematic view illustrating a virtual hybridization process in a flow of frequency analysis of a degree of similarity using a digital DNA chip.

In the virtual hybridization process, a matching process 47 is executed in the nucleic acid information processing device 100 that compares one to one between a nucleic acid fragment list 41 that includes one or a plurality of base sequences 43 identified for all the fragment IDs 42 included in the target, and base sequence information for all probes of a probe base sequence list 44 that includes one or a plurality of base sequences 46 identified for the probe ID 45 for each base. In this case, it is determined over all fragment areas of the probe whether or not each base pair within the target and probe fragment is a match or mismatch, and whether or not a complementary strand should be formed, and the similarity threshold is determined from the values (total matching rate, number of the longest continuous matching bases, the longest continuous matching rate, and the like) of matching conditions within the probe fragment.

The matching process 47 is executed, and for target nucleic acid base sequences that indicate a value of degree of similarity, calculated by comparing one to one using the method described above, between the probe base sequence and the target nucleic acid base sequence, greater than the value of the similarity threshold determined numerically as described above, the nucleic acid information processing device 100 identifies clusters that are collections of fragments for which the base sequence is similar as represented by probe ID 51, and executes an adding process 48 of adding the clusters as cluster members within a virtual hybridization results table 50. Specifically, the nucleic acid information processing device 100 increments a cluster member number 52, adds the target fragment ID 42 as a cluster member fragment ID 53, and adds a target base sequence 43 as a cluster member base sequence 54.

For target nucleic acid base sequences that indicate a value of calculated degree of similarity less than the similarity threshold, the nucleic acid information processing device 100 does not add them to the cluster of the base sequences of the probe of the comparison item of the virtual hybridization results table 50, but executes a change 55 of comparison item (base of a different probe ID as comparison item), and executes the matching process 47 again after changing the probe base sequence to be compared. The target nucleic acid base sequences that have not become cluster members of any probe base sequence even after the matching process 47 has been completed for all the probe base sequences are not added to the virtual hybridization results table 50 by the nucleic acid information processing device 100, but are grouped as reaction negative.

In this way, when the nucleic acid information processing device 100 has finished determining the allocation of the target nucleic acid base sequences that were the subject of comparison to one of the probe base sequence clusters or to a reaction negative group, a change 56 of comparison pairs is executed, and pairs of target nucleic acid base sequence and probe base sequence are newly selected for comparison, and processes such as the matching process 47 are executed. For all the base sequences of the target nucleic acid, when repetition of the above operation has been completed, the nucleic acid information processing device 100 counts the number of base sequences of the target nucleic acid placed in clusters for each probe ID 51 of the virtual hybridization results table 50, and calculates the number of cluster members.

In virtual hybridization using a digital DNA chip, it is just conceivable that it be completed within a few hours at most, even though it greatly depends on the calculation performance of the nucleic acid information processing device. Therefore, there is a high possibility that the processing time can be shortened by using the digital DNA chip.

The information that can be obtained as the final result of the frequency analysis of similar base sequences as described above, in an analysis using a digital DNA chip, includes the number of fragments that belong to a cluster of target fragments having a predetermined degree of similarity to the base sequences of each probe, and information on all the base sequences of all the target fragments obtained in the target preparation stage.

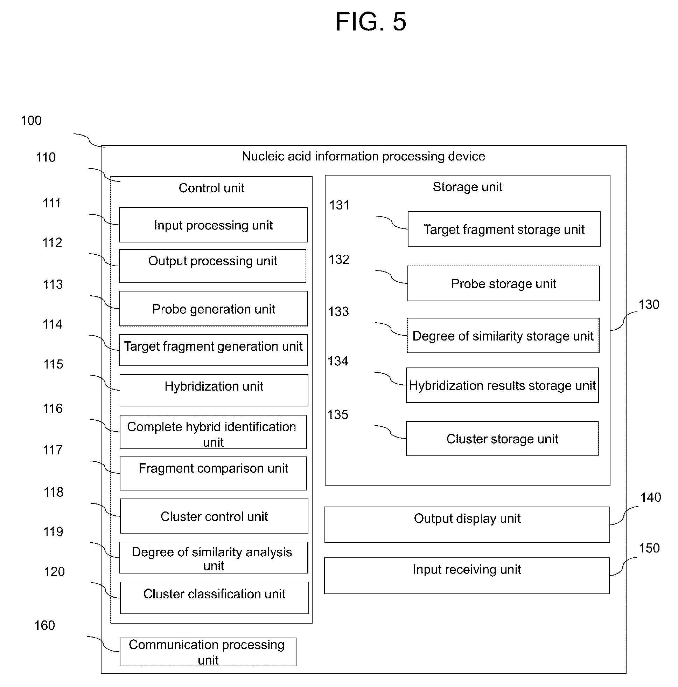

FIG. 5 is a functional block diagram of the nucleic acid information processing device 100. The nucleic acid information processing device 100 includes a control unit 110, a storage unit 130, an output display unit 140, an input receiving unit 150, and a communication processing unit 160. The control unit 110 includes an input processing unit 111, an output processing unit 112, a probe generation unit 113, a target fragment generation unit 114, a hybridization unit 115, a complete hybrid identification unit 116, a fragment comparison unit 117, a cluster control unit 118, a similarity analysis unit 119, and a cluster classification unit 120.

The input processing unit 111 receives input information transmitted from a client terminal (for example, a personal computer loaded with a web browser) (not illustrated), via the communication processing unit 160. However, this is not a limitation, and the input processing unit 111 may receive input information via an input device 101 described below.

The output processing unit 112 transmits output information to the client terminal via the communication processing unit 160. The output information includes target fragment sets, clustering results, probe sets, probe base sequence virtual arrangement lists, virtual hybridization results, comparison analysis results, and the like, as illustrated in FIG. 1. The output processing unit 112 may output output information via an output device 106 described below.

The probe generation unit 113 generates probe information corresponding to the digital DNA chip, using base sequence data. Specifically, the probe generation unit 113 allocates a probe ID as an identifier to the existing digital DNA chip information and base sequence data used as other probes, allocates a probe set ID to which the probe ID belongs, and allocates in sequence the block position corresponding to information that identifies the position on the DNA microarray and the spot position that identifies the position on the block. Then, the probe generation unit 113 stores the strand length (number of bases) of the base sequence data in correspondence to information that identifies the base sequence in a probe storage unit 132 described below. The probe generation unit 113 may execute conversion of the base sequence data provided in a predetermined data format used by existing software packages such as FASTA and basic local alignment search tool (BLAST) into a predetermined data format. FASTA is a software that is capable of searching base sequence databases or amino acid databases using base sequence queries or protein amino acid sequence queries with bioinformatics, and determining the degree of similarity. In FASTA, base sequences are described in a description format known as FASTA format that records base sequence information in plain text. In this embodiment, BLAST refers to an algorithm for executing sequence alignment of DNA base sequences or protein amino acid sequences with bioinformatics. Also, in addition to this common term, a program that executes this algorithm is called BLAST. BLAST is capable of, for example, searching a genome sequence database using an unknown base sequence, and extracting sequence sets with high degrees of similarity, their degrees of similarity, matching percentage, the starting position/finishing position of the matched portion, and the starting position/finishing position of the matched portion on the target base sequence.

The target fragment generation unit 114 stores information on a series of base sequences that constitute a target read by a sequencer or the like in a target fragment storage unit 131 described below, in correspondence with fragment IDs for distinguishing the base sequences from other base sequences. Specifically, a unique identification number or the like is allocated to each base sequence data output from a sequencer and the data is stored in the target fragment storage unit 131.

The hybridization unit 115 executes virtual hybridization. Specifically, the hybridization unit 115 identifies combinations of base sequences of target fragments stored in the target fragment storage unit 131 and probe base sequences stored in the probe storage unit 132 that have a degree of similarity greater than or equal to the threshold, and, for each probe ID, counts the number of target fragments having a degree of similarity greater than or equal to the predetermined threshold and the number of complete hybrids identified by the complete hybrid identification unit 116. In this embodiment, the degree of similarity is the common concept, and is measured from the percentage similarity, the percentage alignment, and the like.

The complete hybrid identification unit 116 extracts and links up matched portion data based on the results of similarity analysis, and identifies base sequences having a degree of similarity greater than or equal to a predetermined value to all base sequences from the starting position to the finishing position of the probe base sequence. Specifically, the complete hybrid identification unit 116 extracts as matched portion data from a degree of similarity storage unit 133 target fragment base sequences that partially match, including target fragment base sequences having a degree of similarity greater than or equal to a predetermined value to the probe base sequence, and links them in sequence based on the matching starting position and finishing position, and if it is possible to link them to the finishing position of the probe base sequence, identifies the linked matched portion data sequence as a complete hybrid.

When the similar portion between single matched portion data and a probe base sequence is the whole probe base sequence, the complete hybrid identification unit 116 identifies the matched portion data as a complete hybrid.

Also, the complete hybrid identification unit 116 is not limited to this type of process, for example, matched portion data that partially matches from the start and finish ends of the probe toward the center may be linked up, and if the matched portion data is linked without a gap, the linked matched portion data set may be identified as a complete hybrid.

In other words, when the similar portion between a single matched portion data and a probe base sequence is the whole probe base sequence, or, when the portion of a plurality of nucleic acid fragments within a target fragment that has been virtually hybridized with the probe base sequence that is similar to a probe base sequence is linked without a gap and the whole of the portion that is similar to the probe base sequence includes the whole of the probe base sequence, the complete hybrid identification unit 116 identifies the matched portion data as a complete hybrid.

The fragment comparison unit 117 executes a target comparison process that compares two different target fragment sets. For example, the fragment comparison unit 117 identifies and outputs difference in the number of cluster members for the results information for the same probe for two different target fragment sets that were virtually hybridized using the same probe set, for example, target fragments extracted from seawater sampled from the same sea area at different times.

The cluster control unit 118 executes a clustering process that classifies target fragments into a predetermined number of cluster sets or less. The cluster control unit 118 groups target fragments within target fragment sets to be classified into clusters in accordance with their degree of similarity, and forms clusters. Specifically, the cluster control unit 118 forms groups by gradually lowering the similarity threshold until the number of received clusters is not more than the upper limit number, and finishes classification into the cluster sets when the upper limit or less is reached. When the similarity threshold has reached the predetermined value (for example, 1.0E+01) by gradually lowering the threshold, the cluster control unit 118 fixes the threshold without lowering the value, and thereafter if the degree of similarity of representative sequences is greater than or equal to the threshold, clusters are merged.

The similarity analysis unit 119 identifies the degree of similarity of two base sequence data. Specifically, the similarity analysis unit 119 identifies the percentage similarity, percentage alignment, and the starting position and the finishing position of the similar portions for two base sequence data according to the complementarity of the base. In other words, in principle, when a complementary base corresponding to a base of a first base sequence data is included in a second base sequence data, it is determined whether or not the bases adjacent to these bases correspond complementarily. This is repeated until a base that does not correspond appears, and, the correspondence is determined in the same way for a different base pair, and the corresponding portion is identified as a similar portion. Combinations for which the length between the starting position and the finishing position of the similar portion is long are deemed to be similar data to that base sequence data. The similarity analysis unit 119 not only determines complementary correspondence of bases, but also determines identity of bases, and determines degree of similarity. In other words, if a series of base sequences included in a first base sequence data (for example, a target) has a predetermined or greater degree of similarity to a series of base sequences included in a second base sequence data (for example, a probe), then the similarity analysis unit 119 deems that the first series of base sequences is a similar portion to the second base sequence data. For identifying the degree of similarity, algorithms such as the existing BLAST algorithm or the like can be used.

The cluster classification unit 120 classifies target fragments into a plurality of clusters in accordance with the degree of similarity. Specifically, the cluster classification unit 120 provides one cluster represented by one fragment from target fragments, and determines whether or not other fragments have a predetermined degree of similarity or greater to the representative fragment of that cluster, and if it has the predetermined degree of similarity or greater, it is classified as belonging to that cluster. If it does not have the predetermined degree of similarity or greater, and if there is another cluster, the cluster classification unit 120 determines the degree of similarity to the representative fragment of that cluster, and if it has the predetermined degree of similarity or greater, it is classified as belonging to that cluster. For fragments that do not have the predetermined degree of similarity or greater to any other cluster, the cluster classification unit 120 provides a new cluster with that fragment as the representative fragment.

The storage unit 130 includes the target fragment storage unit 131, the probe storage unit 132, the degree of similarity storage unit 133, a hybridization results storage unit 134, and a cluster storage unit 135. Also, the storage unit 130 may be a storage device that is installed fixedly in the nucleic acid information processing device 100, or it may be an independent storage device, or the like.

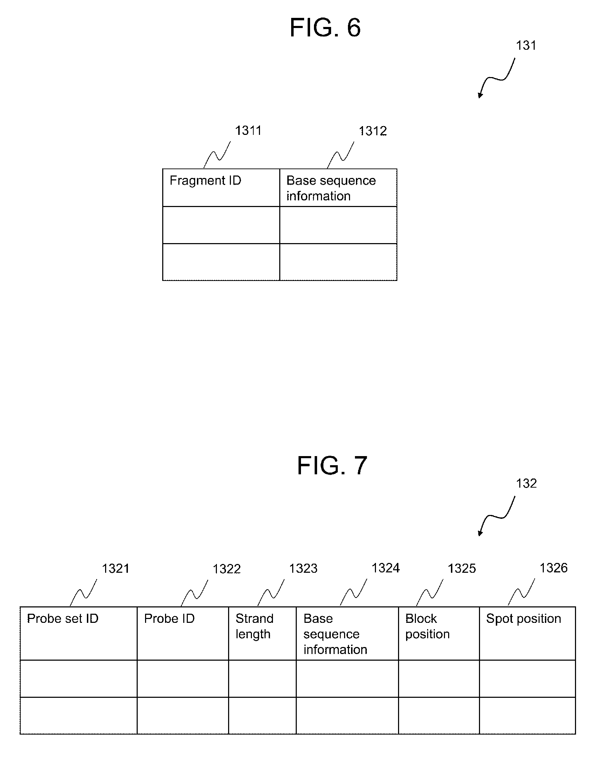

As illustrated in FIG. 6, the target fragment storage unit 131 includes a fragment ID 1311 that includes information for distinguishing the fragment, and base sequence information 1312 which is information on the base sequence of the fragments identified by the fragment ID 1311.

As illustrated in FIG. 7, the probe storage unit 132 includes a probe set ID 1321 that includes information for distinguishing the probe set (digital DNA chip) to which the probe belongs; a probe ID 1322 that includes information for distinguishing the probe base sequence; a strand length 1323 which is the number of bases of the base sequence identified by the probe ID 1322; base sequence information 1324 which is information on the base sequence of the probe identified by the probe ID; a block position 1325 for identifying the schematic arrangement position on the digital DNA chip identified by the probe set ID 1321 of the base sequence of the probe identified by the probe ID; and a spot position 1326 for identifying the detailed arrangement position within the block.

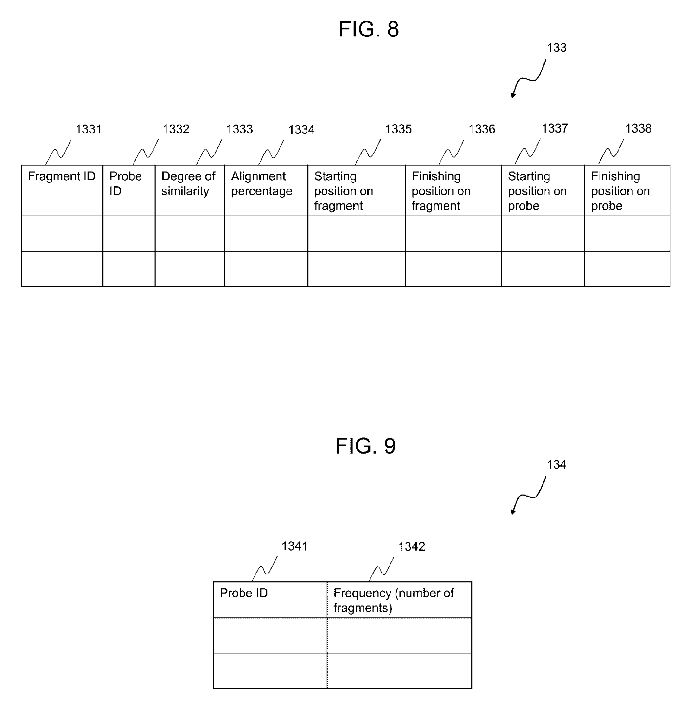

As illustrated in FIG. 8, the degree of similarity storage unit 133 includes a fragment ID 1331 that includes information for distinguishing the base sequence of the fragment that is one of the subjects of similarity analysis; a probe ID 1332 that includes information for distinguishing the base sequence of the probe that is the other subject of the similarity analysis; a similarity percentage 1333 of the base sequence of the fragment identified by the fragment ID 1331 and the base sequence of the probe identified by the probe ID 1332; an alignment percentage 1334; a starting position on the fragment 1335 which is the starting position of the similar portion on the base sequence of the fragment; a finishing position on the fragment 1336 which is the finishing position of the similar portion on the base sequence of the fragment; a starting position on the probe 1337 which is the starting position of the similar portion on the base sequence of the probe; and a finishing position on the probe 1338 which is the finishing position of the similar portion on the base sequence of the probe.

As illustrated in FIG. 9, the hybridization results storage unit 134 is a storage unit that stores information on the results of virtual hybridization in correspondence with a frequency 1342 indicated by the number of fragments with a degree of similarity greater than or equal to the threshold, for each probe ID 1341 that includes information for distinguishing a base sequence of a probe.

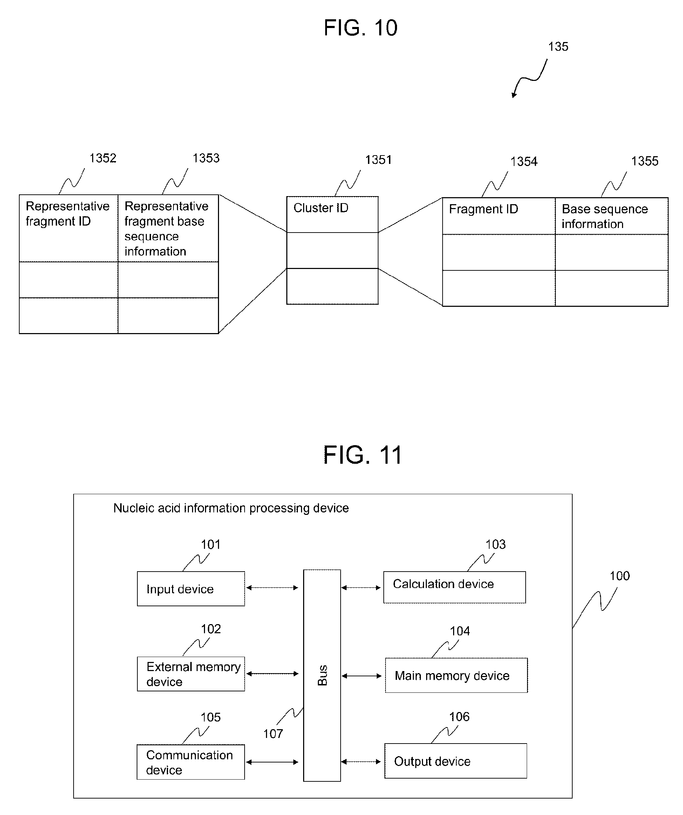

As illustrated in FIG. 10, the cluster storage unit 135 stores a representative fragment ID 1352 that includes information for distinguishing a fragment that represents a cluster, and representative fragment base sequence information 1353 which is information on the base sequence of the representative fragment, for each cluster ID 1351 which includes information for distinguishing a target fragment set classified in the clustering process. Also, the cluster storage unit 135 stores a fragment ID 1354 that includes information for distinguishing fragments belonging to the cluster, and base sequence information 1355 which is information on the base sequence of the fragment, for each cluster ID 1351.

The output display unit 140 outputs various kinds of information from a GUI, a CUI or the like of the nucleic acid information processing device 100. The input receiving unit 150 receives the input of operational information of a GUI or a CUI.

The communication processing unit 160 connects to other devices via a network (not illustrated) or the like, and receives information transmitted from the other connected devices, and transmits information to the other connected devices.

FIG. 11 illustrates a hardware configuration of the nucleic acid information processing device 100 according to this embodiment.

In this embodiment, the nucleic acid information processing device 100 is a dedicated hardware device, for example. However, this is not a limitation, and it may be a computer such as a highly versatile personal computer (PC), a workstation, a server device, various kinds of mobile phone terminals, and a personal digital assistant (PDA).

The nucleic acid information processing device 100 includes the input device 101, an external memory device 102, a calculation device 103, a main memory device 104, a communication device 105, the output device 106, and a bus 107 that connects each of these devices.

The input device 101 is a device that receives inputs from, for example, a keyboard, mouse, touch pen, or other pointing device.

The external memory device 102 is a non-volatile memory device such as a hard disk device and a flash memory.

The calculation device 103 is a calculation device such as, for example, a central processing unit (CPU).

The main memory device 104 is a memory device such as, for example, a random access memory (RAM).

The communication device 105 is a wireless communication device that executes wireless communication via an antenna, or a cable communication device that executes cable communication via a network cable.

The output device 106 is a device that displays, such as a display.

The storage unit 130 of the nucleic acid information processing device 100 is realized by either the main memory device 104 or the external memory device 102.

Also, the input processing unit 111, the output processing unit 112, the probe generation unit 113, the target fragment generation unit 114, the hybridization unit 115, the complete hybrid identification unit 116, the fragment comparison unit 117, the cluster control unit 118, the similarity analysis unit 119, and the cluster classification unit 120 of the nucleic acid information processing device 100 are realized by a program that is processed by the calculation device 103 of the nucleic acid information processing device 100.

This program is stored within the main memory device 104 or the external memory device 102, and, for execution, it is loaded on the main memory device 104, and executed by the calculation device 103.

Also, the output display unit 140 of the nucleic acid information processing device 100 is realized by an output device 106 of the nucleic acid information processing device 100.

Also, the input receiving unit 150 of the nucleic acid information processing device 100 is realized by the input device 101 of the nucleic acid information processing device 100.

Also, the communication unit 160 of the nucleic acid information processing device 100 is realized by the communication device 105 of the nucleic acid information processing device 100.

This completes the hardware configuration of the nucleic acid information processing device 100. The hardware configuration of the nucleic acid information processing device 100, the configuration of the processing units, and the like, are not limited to the above examples, but, for example, may be provided by a configuration of different components using different parts and the like that can be substituted.

For example, the input processing unit 111, the output processing unit 112, the probe generation unit 113, the target fragment generation unit 114, the hybridization unit 115, the complete hybrid identification unit 116, the fragment comparison unit 117, the cluster control unit 118, the similarity analysis unit 119, and the cluster classification unit 120 of the nucleic acid information processing device 100 are classified in accordance with the main processing content, for ease of understanding of the configuration of the nucleic acid information processing device 100. Therefore, the invention according to the present application is not limited by the classification of the constituents or their names. The configuration of the nucleic acid information processing device 100 can be further classified into more detailed constituents in accordance with the processing contents. Also, a single constituent can be classified so that it executes even more processes.

Also, each functional unit of the nucleic acid information processing device 100 may be constructed from hardware (ASIC, GPU, and the like). Also, the process of each functional unit may be executed by a single hardware, or it may be executed by a plurality of hardware.

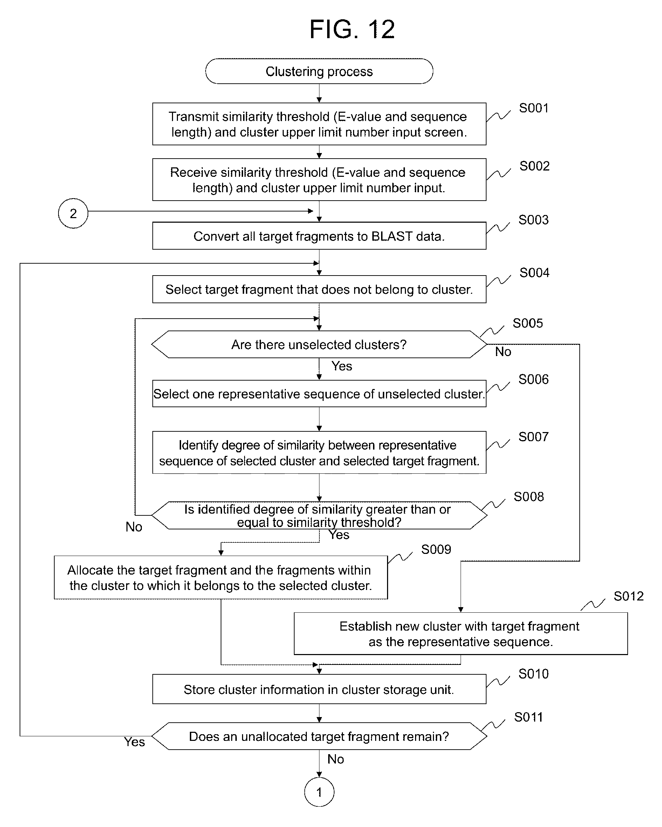

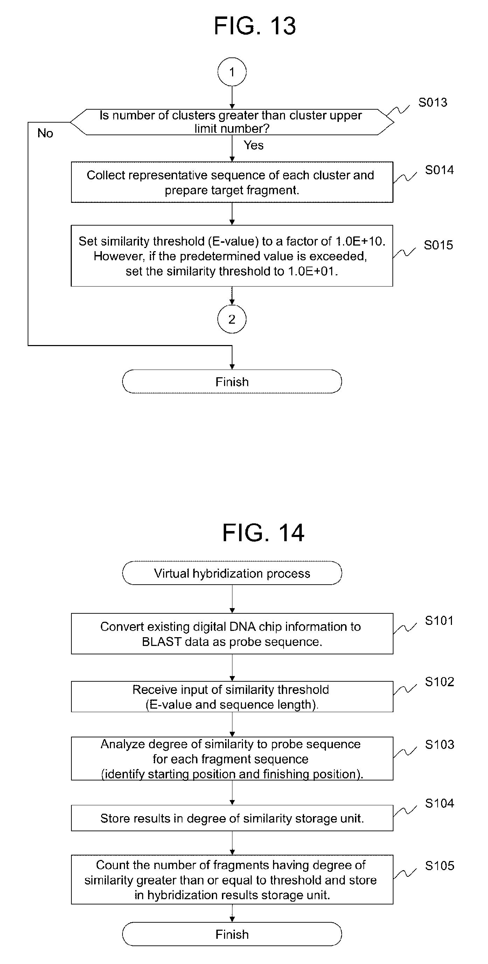

[Description of Operation] Next, the flow of the clustering process executed by the nucleic acid information processing device 100 in this embodiment is described based on FIGS. 12 and 13. FIG. 12 and FIG. 13 are flowcharts illustrating the clustering process. The clustering process is started when a clustering process execution request is received via the network from a client terminal such as a PC (not illustrated) or the like, via a web browser or the like.

First, the cluster control unit 118 configures an input screen of the setting values (similarity threshold and cluster upper limit number) of the cluster. Then, the output processing unit 112 transmits the configured screen to the originator of the execution request (step S001). Specifically, the cluster control unit 118 configures an input screen of the E-value as the similarity threshold, sequence length, and cluster upper limit number, and the output processing unit 112 transmits the configured screen to the originator of the execution request.

The input processing unit 111 receives the input of the similarity threshold, and the cluster upper limit number (step S002). Specifically, the input processing unit 111 receives the E-value, sequence length, and cluster upper limit number as parameters transmitted from the web browser of the client terminal.

The cluster control unit 118 converts all the target fragment base sequence data to be subjected to clustering of which the specification is received by the input processing unit 111 and the like into a data format that can be handled by the BLAST software (step S003). Specifically, the cluster control unit 118 converts all the target fragment base sequence data (for example, in a format that can be processed by FASTA software) to be subjected to clustering of which the specification is received by the input processing unit 111 and the like into a data format that can be processed by the BLAST software.

Then, the cluster classification unit 120 selects a target fragment that does not belong to a cluster (step S004). Specifically, the cluster classification unit 120 selects a single target fragment that does not belong to any cluster and that has not been subjected to the cluster classification process from the target fragment set in a data format that can be processed by the FASTA software.

Next, the cluster classification unit 120 determines whether or not there is an unselected existing clusters (step S005). Specifically, the cluster classification unit 120 determines whether or not an unselected cluster remains from the existing clusters formed by the clustering process.

If there are unselected existing clusters (YES at step S005), the cluster classification unit 120 identifies the unselected existing clusters, and selects the representative sequence of the cluster (step S006).

Then, the similarity analysis unit 119 identifies the degree of similarity between the selected representative sequence and the selected target fragment (step S007). Specifically, the similarity analysis unit 119 identifies the degree of similarity (similarity percentage, alignment percentage, starting position and finishing position of the similar portion on the target fragment, and starting position and finishing position of the similar portion on the probe base sequence) of both of the sequences, in the same way as the BLAST software, and stores it in the degree of similarity storage unit 133. In this process, the similarity analysis unit 119 identifies the degree of similarity using the similarity threshold received in step S002.

Then, the cluster classification unit 120 determines whether or not the degree of similarity identified is greater than or equal to the similarity threshold (step S008). Specifically, the cluster classification unit 120 determines whether or not the degree of similarity between the selected representative sequence and the selected target fragment identified in step S007 is greater than or equal to the similarity threshold received in step S002.

If it is not greater than or equal to the similarity threshold (NO at step S008), the cluster classification unit 120 returns the control to step S005 in order to identify the degree of similarity to the representative fragment of another cluster.

If it is greater than or equal to the similarity threshold (YES at step S008), the cluster classification unit 120 allocates the target fragment and the fragment within the cluster to which it belongs to the cluster to which the selected representative sequence belongs (step S009). More specifically, if the target fragment that was compared for the degree of similarity belongs to a cluster, the cluster classification unit 120 allocates all the fragments belonging to that cluster and the target fragment to the existing cluster that was represented by the representative sequence that was compared for the degree of similarity. In this case, for the target fragment whose allocation was changed, the cluster classification unit 120 deletes that target fragment from the cluster to which the target fragment belonged.

Then, the cluster classification unit 120 stores the cluster information in the cluster storage unit 135 (step S010). Specifically, the cluster classification unit 120 stores information regarding all the fragments that were allocated in step S009 in the fragment ID 1354 and base sequence information 1355 of the cluster storage unit 135. If there is no fragment that is newly allocated, it is not necessary for the cluster classification unit 120 to store information in the cluster storage unit 135, so no particular process is executed.

Then, the cluster classification unit 120 determines whether or not an unallocated target fragment remains (step S011). Specifically, the cluster classification unit 120 determines whether or not a target fragment that is not allocated to any cluster remains in the target fragment set.

If an unallocated target fragment remains (YES at step S011), the cluster classification unit 120 returns the control to step S004.

If an unallocated target fragment does not remain (NO at step S011), the cluster control unit 118 proceeds to step S013 described below.

In the decision at step S005 as described above, if there is no unselected existing clusters (NO at step S005), the cluster classification unit 120 establishes a new cluster with the target fragment as the representative sequence (step S012). Specifically, the cluster classification unit 120 stores information regarding the target fragment in the representative fragment 1352 and the representative fragment base sequence information 1353.

Then, the cluster control unit 118 determines whether or not the number of clusters is greater than the cluster upper limit number (step S013). Specifically, the cluster control unit 118 counts the number of cluster IDs 1351 stored in the cluster storage unit 135, and compares it with the cluster upper limit number received as input in step S002. If the number of clusters is equal to or less than the cluster upper limit number (NO at step S013), the cluster control unit 118 terminates the clustering process.

If the number of clusters is greater than the cluster upper limit number (YES at step S013), the cluster control unit 118 collects the representative sequence of each cluster and creates target fragments (step S014).

Then, the cluster control unit 118 sets the E-value which is the similarity threshold to a factor of 1.0E+10 (step S015), and returns the control to step S003. By doing so, it is possible to determine the degree of similarity between cluster representative sequences with relaxed degree of similarity, and integrate in order to keep the number of clusters equal to or less than the upper limit number. When the E-value is set to a factor of 1.0E+10, if the E-value exceeds the value 1.0E+01 which is set in advance, the cluster control unit 118 sets the E-value to 1.0E+01, and returns the control to step S003.

This ends the flow of the clustering process. Using the clustering process, the nucleic acid information processing device 100 can cluster target fragments based on the specified similarity threshold and the cluster upper limit number. In other words, a target can be classified so that the degree of similarity of the target is not less than a predetermined value. The clusters obtained by the clustering process of this embodiment have a homology interval between representative sequences that is substantially constant. In this case, when a target that includes several types of organisms and the like is classified into clusters, as a result of the law of large numbers, cluster sets are obtained with an approximately constant homology interval. This is effective for preparing probes with a constant degree of similarity, and the like, when executing tests to determine the trend of the variation with time of a configuration of base sequence, with a target that includes organisms that are configured from unknown base sequences, and the like.

Next, the flow of the virtual hybridization process executed by the nucleic acid information processing device 100 according to this embodiment is described based on FIG. 14. FIG. 14 is a flowchart illustrating the virtual hybridization process. The virtual hybridization process is started when a virtual hybridization execution request is received via a network from a client terminal such as a PC (not illustrated) via a web browser or the like.

First, the probe generation unit 113 converts existing digital DNA chip information into BLAST data as the probe sequence (step S101). Specifically, the probe generation unit 113 allocates a probe ID as identifier to the existing digital DNA chip information and base sequence data used as other probes, allocates a probe set ID to which the probe ID belongs, and allocates a block position corresponding to the information that identifies the position on the DNA microarray, and a spot position that identifies the position on the block. Then, the probe generation unit 113 stores the strand length (number of bases) of the base sequence data in correspondence with the information for identifying the base sequence in the probe storage unit 132 described below. Then, the probe generation unit 113 converts the existing digital DNA chip information and the base sequence data used as the other probe into a predetermined data format used in the BLAST software package.

Then, the input processing unit 111 receives input of the similarity threshold (E-value and sequence length) (step S102). Specifically, the output processing unit 112 transmits a predetermined similarity threshold input screen to a client terminal for display, and the input similarity threshold is received by the input processing unit 111.

Then, the hybridization unit 115 analyzes the degree of similarity of the probe sequence (for example, the representative sequence of each cluster) for each fragment sequence, based on information stored in advance in the target fragment storage unit 131 by the target fragment generation unit 114 (step S103). Specifically, the hybridization unit 115 delegates the processing to the similarity analysis unit 119 for all combinations of target fragment base sequence and probe base sequence, to identify the degree of similarity and the starting position and the finishing position of similar portions on the target fragment base sequence and probe base sequence.

Then, the hybridization unit 115 stores the analyzed degree of similarity results in the degree of similarity storage unit 133 (step S104).

From the degree of similarity analysis results, the hybridization unit 115 counts the number of fragments having a degree of similarity greater than or equal to the similarity threshold for each probe, and stores the result in the hybridization results storage unit 134 (step S105).

That completes the flow of the virtual hybridization process. As a result of the virtual hybridization process, the nucleic acid information processing device 100 can count the number of target fragments having a degree of similarity greater than or equal to the specified similarity threshold, for each probe base sequence. In other words, when a probe base sequence is the representative base sequence of a cluster, it is possible to identify the frequency of the base sequence included in the target for each cluster. Also, as a result of the virtual hybridization process, the nucleic acid information processing device 100 can identify the degree of similarity and the parts thereof for all combinations of target and probe. In step S105 of the above process, the hybridization unit 115 may count a series of base sequences for each probe that have been deemed to be complete hybrids in a complete hybrid identification process described below, and store the result in the hybridization results storage unit 134. In this way, even when the fragment is more divided than the probe sequence, it is possible to obtain an appropriate frequency.

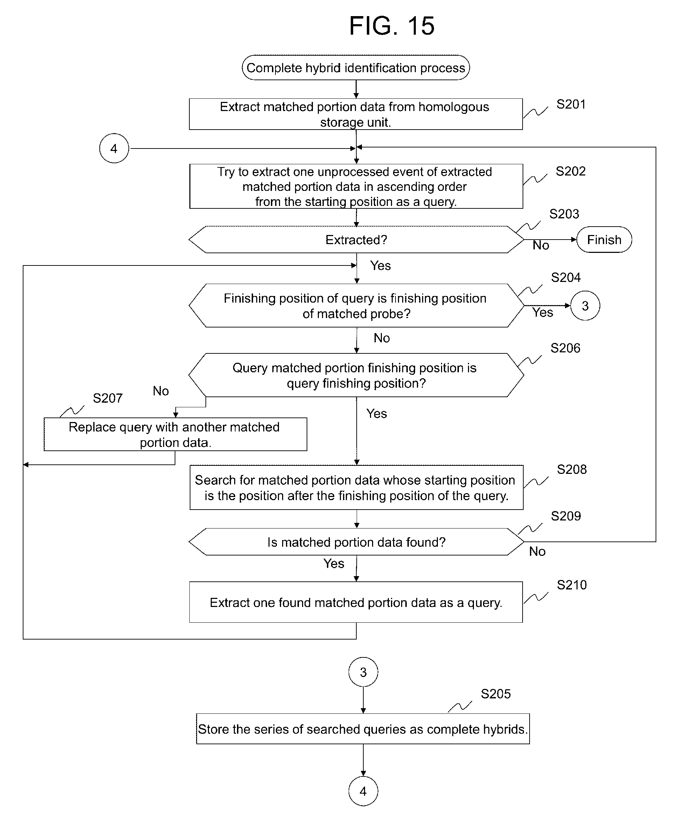

Next, the flow of the complete hybrid identification process executed by the nucleic acid information processing device 100 according to the present embodiment is described based on FIG. 15. FIG. 15 is a flowchart illustrating the complete hybrid identification process. The complete hybrid identification process is executed using the results of the virtual hybridization process, so it is started after the virtual hybridization process. Also, when a complete hybrid identification process execution request is received via a network from a client terminal such as a PC (not illustrated) or the like, via a web browser or the like, the process is started.

First, the complete hybrid identification unit 116 extracts matched portion data from the degree of similarity storage unit 133 (step S201). The matched portion data includes completely matched portion data. In this embodiment, matched portion data is target fragment base sequence data of a target fragment having a similar portion (in other words, a similar portion having a predetermined degree of similarity to a probe sequence) that has a value of degree of similarity to the probe sequence greater than or equal to a predetermined value. Also, completely matched portion data is target fragment base sequence data of a target fragment having similar portions only whose degree of similarity exhibits a complete match to the probe sequence.

The complete hybrid identification unit 116 extracts as a query from the extracted matched portion data an unprocessed event in ascending order from the starting position on the probe (step S202). Specifically, the complete hybrid identification unit 116 sorts the matched portion data extracted in step S201 in ascending order of the starting position on the probe 1337, and attempts to extract as a query an unprocessed event from the matched portion data that has the same starting position on the probe 1337 as the sorted starting matched portion data and the starting position of the similar portion. In this case, in addition, the complete hybrid identification unit 116 extracts only target fragments (in other words, completely matched portion data is included) for which the finishing position (in other words, the finishing position on the fragment 1336) of the similar portion of the matched portion data and the finishing position (in other words, the position of the end of the fragment) of the matched portion data match.

The complete hybrid identification unit 116 determines whether or not a query has been extracted (step S203). If a query has not been extracted (NO at step S203), the complete hybrid identification unit 116 terminates the complete hybrid identification process.

If a query has been extracted (YES at step S203), the complete hybrid identification unit 116 determines whether or not the finishing position (finishing position on the fragment 1336) of the similar portion of the base sequence of the query is the finishing position (finishing position on the probe 1338) of the matched probe (step S204).

If it is the probe finishing position (YES at step S204), the complete hybrid identification unit 116 stores the searched series of queries in a predetermined area of the storage unit 130 as a complete hybrid (step S205). Then, the complete hybrid identification unit 116 returns the control to step S202.

If it is not the probe finishing position (NO at step S204), the complete hybrid identification unit 116 determines whether or not the finishing position (in other words, the finishing position on the fragment 1336) of the similar portion of the matched portion data of the query is the finishing position (in other words, the position of the end of the fragment) of the matched portion data (step S206), and if it is not the finishing position of the matched portion data, then it selects as a query another matched portion data that is different from the matched portion data searched in step S206 (step S207), and returns the control to step S204. If it is the finishing position of the matched portion data, the complete hybrid identification unit 116 searches the matched portion data with a starting position that is the next position after the finishing position of the query (step S208). In this case, the complete hybrid identification unit 116 further extracts only target fragments (in other words, completely matched portion data is included) for which the starting position of the similar portion (in other words, the starting position on the fragment 1335) of the matched portion data is the starting position (in other words, the position of the start of the fragment) of the matched portion data.

Then, the complete hybrid identification unit 116 determines whether or not matched portion data was found in the search results (step S209). If no matched portion data was found (NO at step S209), the complete hybrid identification unit 116 returns the control to step S202.

If matched portion data was found (YES at step S209), the complete hybrid identification unit 116 extracts the matched portion data as a query (step S210). Then, the complete hybrid identification unit 116 returns the control to step S204.

That completes the flow of the complete hybrid identification process. As a result of the complete hybrid identification process, when the nucleic acid information processing device 100 combines one or a plurality of combinations of matched portion data (including complete matched portion fragments for which the similar portion extends throughout the total length of the fragment) to identify base sequences having a degree of similarity greater than or equal to a predetermined value with respect to all the base sequences from the probe starting position to the finishing position. In other words, even when the base strand length of the target fragment is short, it is possible to maintain a constant accuracy of the virtual hybridization. Also, the complete hybrid identification process is not limited to the above, for example, for a portion of the similar portion on the probe, if a plurality of target fragments having overlapping similar portions is combined, then base sequences that completely match the probe may be identified as complete hybrids. In this way, it is possible to allow complete hybrids of a plurality of target fragments in which a portion of the similar portions is overlapping (in other words, they have an overlapping portion).

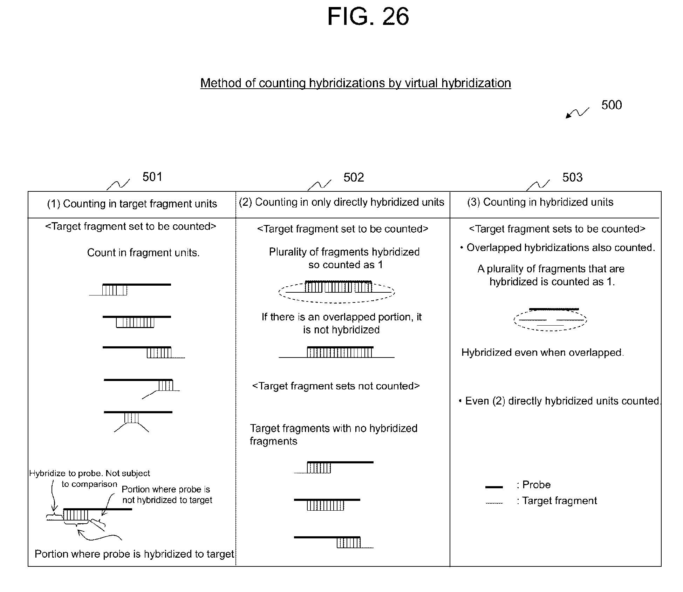

This point is described using FIG. 26. FIG. 26 illustrates methods of counting targets in the virtual hybridization process according to this embodiment.

In this embodiment, three target counting methods are supposed. The first is a counting method in target fragment units 501, as described above. This is a method of counting in hybridized target fragment units, in other words, a method of simply counting the number of target fragments that include similar portions. The second is a counting method in directly linked units 502, as described above. This is a method of counting the number of sets of a plurality of target fragments in which the similar portions of the target fragments are linked with no gap. For example, this is a method in which if the similar portions of three target fragments are linked with no gap, the set of three target fragments is counted if it is similar to the probe. The third is a counting method in linked units 503, as described above. This is a method of counting the number of sets of a plurality of target fragments in which a portion of the similar portions of the plurality of target fragments is linked. Unlike the counting method in directly linked units 502, in this method, when target fragments are linked, sets are counted even when a portion of the similar portions is overlapped. In other words, the counting method in directly linked units 502 is a counting method that permits a certain amount of error.

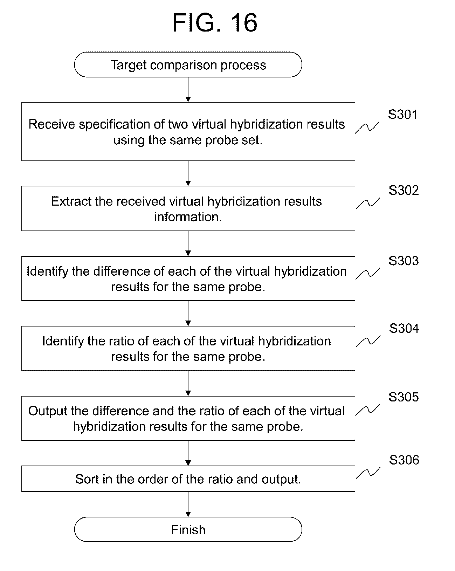

Next, the flow of the target comparison process executed by the nucleic acid information processing device 100 according to this embodiment is described based on FIG. 16. FIG. 16 is a flowchart of the target comparison process. The target comparison process is a process executed using the results of the virtual hybridization process, so it is started after the virtual hybridization process. Also, the process is started when a target comparison process execution request is received via a network from a client terminal such as a PC (not illustrated) or the like, via a web browser or the like.

First, the input processing unit 111 receives the specification of two virtual hybridization results using the same probe set (step S301). Specifically, the input processing unit 111 receives the specification of the hybridization results storage unit 134 for two virtual hybridization results using the same probe set, in other words, for a set of different target fragments in the same probe set in which virtual hybridization was executed.

The fragment comparison unit 117 extracts information on the received virtual hybridization results (step S302). Specifically, the fragment comparison unit 117 reads out the information of the two received hybridization results storage unit 134.

Then, the fragment comparison unit 117 identifies the difference in the virtual hybridization results for each of the same probe (step S303). Specifically, the fragment comparison unit 117 identifies each number of cluster members for the common probes, and calculates the difference by subtracting one from the other.

The fragment comparison unit 117 identifies the ratio of the virtual hybridization results for each of the same probe (step S304). Specifically, the fragment comparison unit 117 identifies each number of cluster members for the common probes, and calculates the ratio of one to the other.

The output processing unit 112 outputs the difference and the ratio of the virtual hybridization results for each of the same probe (step S305). Specifically, the output processing unit 112 outputs the difference and the ratio of the number of cluster members determined in step S304 and step S305, for the common probes.

Also, the output processing unit 112 outputs the virtual hybridization results for each of the same probe, arranging them in order of ratio (step S306). Specifically, the output processing unit 112 outputs the ratio of the number of cluster members for the common probes in descending order. Naturally, the output processing unit 112 may output the ratio of the numbers of cluster members arranged in ascending order.

That completes the flow of the target comparison process. As a result of the target comparison process, it is possible to simply compare components between two targets. In the target comparison process, it is possible to compare the frequency analysis results of a plurality of similar base sequences, including any combination of virtual hybridization results, imported DNA chip experiment data, or a combination of virtual hybridization results and DNA chip experiment data. As discussed above, the results of the virtual hybridization process provide information as numerical data, namely, the number of fragments for each probe, and the results for the DNA chip experiment data provide relative values of the fluorescent intensity of fluorescent dye, so the two cannot be simply compared. Therefore, in the target comparison process, for virtual hybridization results, the fragment comparison unit 117 may obtain the numerical count of each probe as a proportion of the total number of fragments, and for the DNA chip experiment data results, may obtain the fluorescent intensities of each probe as a proportion of the fluorescent intensity of the total chip, and compare the two.

That completes the description of the first embodiment according to the invention of the present application. According to the first embodiment of the invention of the present application, it is possible to virtually hybridize a probe base sequence and a target base sequence. Also, it is possible to configure clusters from target base sequences as a result of the clustering process, and to create a probe base sequence based on the clusters. Also, it is possible to compare hybridization results for the same probe, and to indicate their differences. For example, for target fragments extracted from seawater sampled from the same sea area at different times, it is possible to output the change in the number of cluster members for the same probe. This is capable of clearly indicating changes with time in the configuration of nucleic acid base sequence contained in the same sea area, so, for example, taking statistics on the changes in specific components, and using them to make predictions on the symptoms of occurrence of specific abnormalities (red tide, and the like) can be considered.

According to the first embodiment of the invention of the present application, by determining the base sequence of all the nucleic acid of the subject of the analysis, and using it to analyze the types and frequency of nucleic acid base sequence included in the material, all by information analysis on a computer, it is not necessary to obtain the target fragment base sequence information again when analyzing the next time, unlike when frequency analysis of similar base sequences is executed by tests using a DNA microarray.

Also, the possibility remains that an experimental error is produced in the process of determining the base sequence, but there are no errors in the frequency analysis of similar base sequences based on the determined base sequence information, so highly accurate data with 100% reproducibility can be obtained for the results obtained from frequency analysis of similar base sequences obtained by virtual hybridization, as long as the same combination of probe base sequence list and target fragment base sequence set is used.

Also, in frequency analysis of similar base sequences by testing using a DNA microarray, the GC content and the individual sequence properties of the probe DNA are different, so the degree of similarity in the actual hybridization varies with each probe even within the same microarray, and it is extremely difficult to correct for this difference. However, by executing virtual hybridization completely by information analysis on a computer only, as described above, it is possible to determine the degree of similarity of probe base sequences and target nucleic acid fragment base sequence for any defined number of matching percentage of the target fragment base sequence with respect to total probe base sequence and/or the length of matching base sequence of the target fragment base sequence with respect to the probe base sequence.

Also, by linking together nucleic acid fragments included in one or a plurality of targets, and taking as positive as a complete virtual hybridization is obtained only when a result in which a predetermined degree of similarity or greater is obtained across the whole probe base sequence, it is possible to execute analysis with a higher degree of similarity with respect to the probe base sequence, by analyzing that frequency.

Of these, in particular, analysis to determine whether or not it is possible to link together nucleic acid fragments included in a plurality of targets having a degree of similarity across the whole probe base sequence requires a large quantity of complex information processing, so it was not executed as a conventional experiment, but it is possible to simply execute such analysis. For example, this analysis method is extremely effective for executing analysis of the types and frequencies of nucleic acid included in targets having a degree of similarity greater than or equal to a fixed value across specific genes or whole regions.

Also, in tests using a DNA microarray, the base sequence of the target fragment is not known, but, in analysis by digital DNA chip, all the base sequences of all the target fragments are determined at the stage of the preparatory operation, so a probe base sequence list can be produced infinitely in any condition from the list of base sequences of the nucleic acid fragments included in the target. Therefore, if these are used, virtual hybridization can be executed any number of times with respect to a new probe sequence list and always having 100% reproducibility. This is a great advantage compared with tests using a DNA microarray in which, in each test, target nucleic acid is consumed, so there is a limit to the number of times that a test can be executed using a DNA microarray having new probe base sequence.

Also, clustering is executed by analyzing one fragment at a time in sequence to determine whether or not its degree of similarity is greater than or equal to a predetermined value with respect to the nucleic acid fragment that is used as the standard, and when the degree of similarity is greater than or equal to the predetermined value, a cluster is identified, so it is possible to greatly reduce the number of times the operation to determine the degree of similarity is executed for clustering, compared with determining by round robin whether or not the degree of similarity is greater than or equal to the predetermined value between all the nucleic acid fragment base sequences included in the target, so the time required for clustering is shortened, and it is possible to reduce the computer capacity required for clustering.

Also, when classifying clusters, it is possible to optionally determine the cluster upper limit number up to the number of fragments included in the target as the maximum value. By this determining method of the upper limit value, it is possible to increase or decrease the size of clusters. As a result, when this cluster classification method is used in metagenomic analysis, for example, by determining the cluster upper limit number and executing the classification, it is possible to increase or decrease the level of classification of cluster, such as clusters of size equivalent to classification of species, clusters of size equivalent to classification of genus, and clusters of size equivalent to classification of family, so that the summary of classification results of the analysis are easy to understand.

Also, if a probe base sequence list is to be prepared from a nucleic acid fragment base sequence list included in a target, under any condition, a new probe base sequence list can be prepared rapidly with a small capacity computer.

Also, as described above, if analysis is executed by virtual hybridization for the types and frequencies of nucleic acid included in a plurality of targets using the same probe base sequence list, and the number of cluster members of each probe is compared between the plurality of targets, to extract clusters with different numbers of cluster members between the targets, for all the information of the virtual hybridization analysis, the difference can be analyzed with 100% reproducibility for the types and frequencies of nucleic acid between targets. This makes up for the disadvantage of analysis by testing using DNA microarrays that it is not possible to obtain 100% reproducibility for the hybridization results and comparison data between a plurality of targets based on these results.

Also, if the method of comparative analysis of types and frequencies of nucleic acid included in a plurality of targets using virtual hybridization is used in the analysis of targets sampled in a time series, it is possible to determine the changes in the numbers of cluster members of each probe with 100% reproducibility, so it is possible to increase the accuracy of determining the present status of the changes and predicting trends for the future, compared with analysis using DNA microarrays.

Also, analysis using digital DNA chips can be used for analyzing any of individual bion, parts, tissues, and cells, or their combinations. In addition, with a digital DNA chip, the list of base sequences of all the nucleic acid fragments included in the target is prepared for all targets, so integration is easy. Therefore, by integrating analysis results, such as by integrating the analysis results for a plurality of cells and reanalyzing as a tissue or part, it is possible to execute digital DNA chip analysis at a new step.

Also, comparison of the analysis results of digital DNA chip analyses can be used for analysis of a plurality of bion, parts, tissues, cells, or mixtures thereof. In this case, the reproducibility of the comparison analysis results is 100%.

Also, comparison of the analysis results of digital DNA chip can be used for analysis of liquids, solids, and gases that include biological material containing a plurality of bion, parts, tissues, cells, or mixtures thereof. For example, this type of analysis can be applied to structural analysis of bacterial populations living in seawater in a specific sea area or analysis of their changes, and the like. In this case, also the reproducibility of the comparison results is 100%.

In the above, an embodiment of the present invention was specifically described based on the embodiment, but the present invention is not limited to this, and various changes can be made without deviating from the intent of the present invention.

For example, in the embodiment as described above, the degree of similarity analysis process is executed by existing technology such as the BLAST software, but this is not a limitation. For example, the analysis of the degree of similarity may be executed using another algorithm that is capable of executing degree of similarity analysis. By doing so, the analysis can be executed more flexibly. Also, in the embodiment as described above, the degree of similarity analysis results and the virtual hybridization process results are mainly stored in a database or the like, but the progress or results may be successively displayed on a screen, in accordance with the progress of the clustering process or the virtual hybridization process. By so doing, the progress of the process can be seen visually, so it is easy to predict the time required to complete the process, and the like.

For example, in the embodiment as described above, the nucleic acid information processing device 100 is a device with dedicated hardware, but this is not a limitation, and it may be mounted on a sequencer that can read genetic information, for example. In this way, the hardware device can be simplified.

In the embodiment as described above, the nucleic acid information processing device 100 is not only the object of transaction as a device, but can also be the object of transaction in program component units that realize the operation of the device.

### Examples

In the following, an example of the present invention is specifically described. However, the present invention is not limited to this example.

In this example, an analysis is executed in which the base sequence of microbial DNA of seawater is determined using a DNA sequencer, a probe base sequence list is prepared by clustering using the information, and virtual hybridization of all the base sequences of the microbial DNA in the seawater determined by the DNA sequencer and the probe base sequence list is executed. In addition, a comparison is executed of the results of the virtual hybridization executed in the digital DNA chip named "Y022L08_C10000_chip" for each of the target fragment sets of the microbial DNA in two sets of seawater.

First, the operation to obtain data on the target base sequence from the DNA base sequence of all the microbes in the seawater of a specific sea area was executed. From about 21 liters of seawater filtered with a glass fiber filter paper (produced by Whatman, free of binding agent, pore size: 0.7 µm) sampled at the coast near Fukuura, Kanazawa-ku, Yokohama City, 20 µg of genome DNA was extracted using a water DNA isolation kit (produced by MO BIO Laboratories, Inc., UltraClean with 0.22 µm water filter kit).

The genome DNA solution was concentrated by a factor of about 3 using Microcon YM-100 (produced by Millipore Corporation), and at a final concentration of 10 µg/mL the RNA was digested in one hour at room temperature using Ribonuclease (DNase free) Solution (produced by Nippon Gene Co., Ltd.).

Next, an equal quantity of Phenol / Chloroform / Isoamyl alcohol (25:24:1, produced by Nippon Gene Co., Ltd.) was added to the genome DNA solution, and after mixing gently for five minutes at room temperature, the solution layer was separated by centrifugation at 20,400 g at 20°C for five minutes using a microcentrifuge, the aqueous layer solution was recovered, and the operation was executed twice. An equal quantity of chloroform (reagent grade, produced by Wako Pure Chemical Industries, Ltd.) was added to this aqueous layer solution, and after mixing gently for five minutes at room temperature, the solution layer was separated by centrifugation at 20,400 g and 20°C for five minutes using a microcentrifuge, the aqueous layer solution was recovered, and the operation was executed twice.

To this aqueous layer solution, 3M sodium acetate (produced by Nippon Gene Co., Ltd.) was added to give a final concentration of 0.2 M and mixed, then, ethanol (reagent grade, 99.5%, produced by Wako Pure Chemical Industries, Ltd.) was added at double the quantity of the aqueous layer solution, and ethanol precipitation was executed at -20°C for two hours. Centrifugation was executed at 20,400 g at 4°C for 20 minutes using a microcentrifuge to recover the genome DNA, it was washed with 500 µL of ethanol (reagent grade, 99.5%, produced by Wako Pure Chemical Industries, Ltd.) diluted to a final concentration of 70% with distilled water (deionized, sterile) produced by Nippon Gene Co., Ltd., and dried.

The genome DNA obtained was dissolved in 100 µL TE (produced by Nippon Gene Co., Ltd., pH 8.0), and 5 µg of genome DNA was obtained. Using 500 ng of this, a target for determining the base sequence was prepared in accordance with the manual for the sequencer GS FLX Titanium by Roche Diagnostics K.K., then using the GS FLX Titanium, the base sequence of all the DNA fragments in the target was determined. With regards to the base sequence, the entire assay surface of the sequencer was partitioned into two sections, and the analysis results obtained were named 1.GAC.454Reads.fna and 2.GAC.454Reads.fna. Together these were the sequence results at the maximum limit at one time using the GS FLX Titanium.

As a result, for 1.GAC.454Reads.fna, base sequence data of 293,720,669 bases was obtained for 661,821 fragments, and for 2.GAC.454Reads.fna, base sequence data of 261,548,803 bases was obtained for 619,241 fragments, for a total base sequence data of 555,269,472 bases obtained for 1,281,062 fragments, as base sequences satisfying the base sequence quality recommended by Roche Dioagnostics K.K.

In order to analyze this data with the nucleic acid information processing device 100 using a digital DNA chip, the data was imported into the nucleic acid information processing device 100, then, first, in order to prepare a probe base sequence list for virtual hybridization, the clustering process was executed by the BLAST method using only data for which the number of bases in one fragment was 100 or greater among all the data, and the probe generation process was executed. It is possible to prepare a set of probe base sequences by this method because all the nucleic acid base sequence data included in the target is obtainable in the method, and this is a major advantage of the method of analysis using a digital DNA chip.

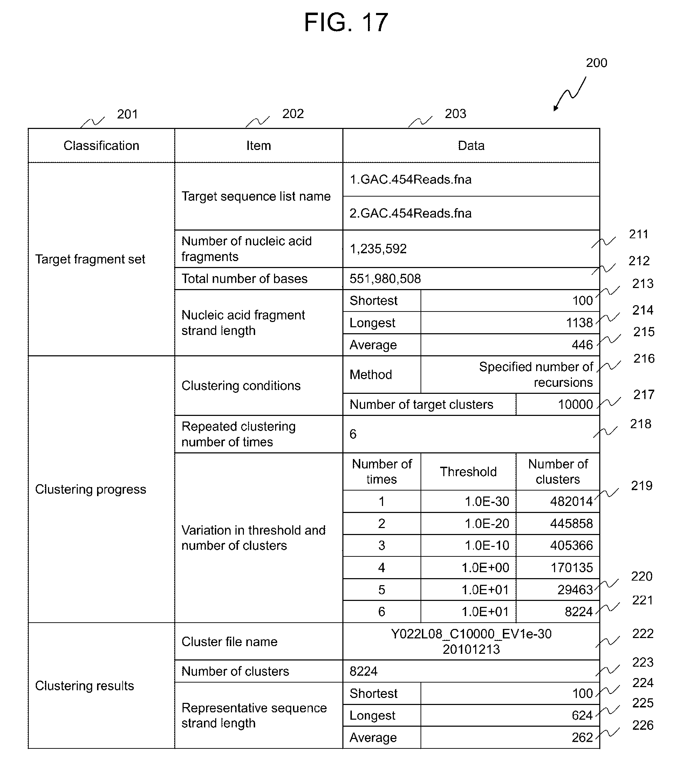

FIGS. 17 to 20 illustrate examples of the output during the clustering process. First, the base sequences of 551,980,508 bases in 1,235,592 fragments for both 1.GAC.454Reads.fna and 2.GAC.454Reads.fna were clustered with a target number of 10,000 clusters, and the results in table 200 shown in FIG. 17 were obtained.

Table 200 is configured to include target fragment sets 201, items 202, and data 203 as the major table items, and number of nucleic acid fragments 211, total number of bases 212, the shortest strand length of nucleic acid fragment 213, the longest strand length of nucleic acid fragment 214, average strand length of nucleic acid fragment 215, method as clustering condition 216, number of target clusters 217, number of repeated clustering times 218, the variation of number of clusters with similarity threshold 219 to 221, cluster file names 222, number of clusters 223, the shortest representative sequence strand length 224, the longest representative sequence strand length 225, average representative sequence strand length 226, and the like. The cluster control unit 118 acquires the required values for display by the output processing unit 112.

In this example, the E-value threshold was first set to 1.0E-30 and clustering was executed by the BLAST method, and the number of clusters obtained was 482,014. Then, the E-value threshold was increased to 1.0E-20, and clustering of the cluster representative sequences was executed. As a result, the number of clusters obtained was 445,858. This was greater than the target upper limit of 10,000, so then, the E-value threshold was reduced to 1.0E-10, 1.0E+00, and 1.0E+01, and the clustering was repeated. However, the number of clusters obtained was 29,463, so it was not reduced below the target upper limit. Therefore, the value of the E-value was fixed at the value 1.0E+01, and clustering was repeated until the number of clusters obtained was 10,000 or less. Clustering was executed for a total of six times, the number of clusters obtained was 8,224, and the cluster set for this clustering result was named "Y022L08_C10000".

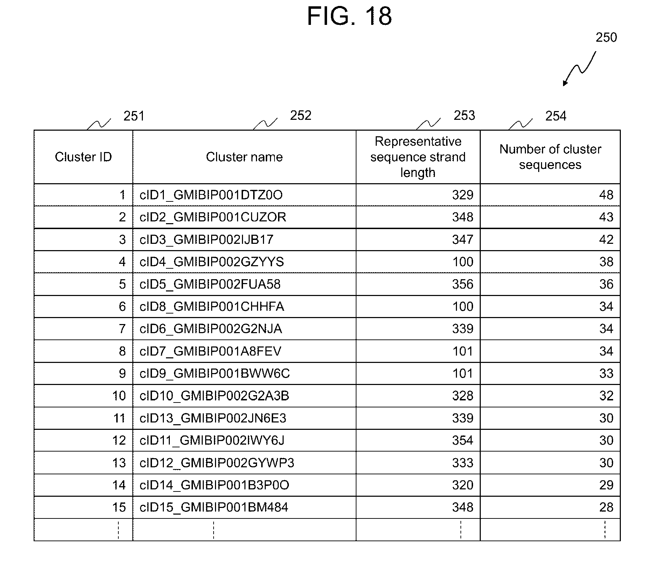

The clusters included in this cluster set are shown in Table 250 which shows a summary for each cluster name 252 shown in FIG. 18. Table 250 includes the cluster name 252, the representative sequence strand length 253, and the number of cluster sequences 254, for each cluster ID 251. Therefore, it is possible to list the representative sequence strand length 253 and the number of fragments belonging to each cluster (the number in the column of number of cluster sequences 254, which corresponds to the number of linked fragments). In this example, the number of clusters is large, so, in FIG. 18, only a portion of the Table 250 is shown.

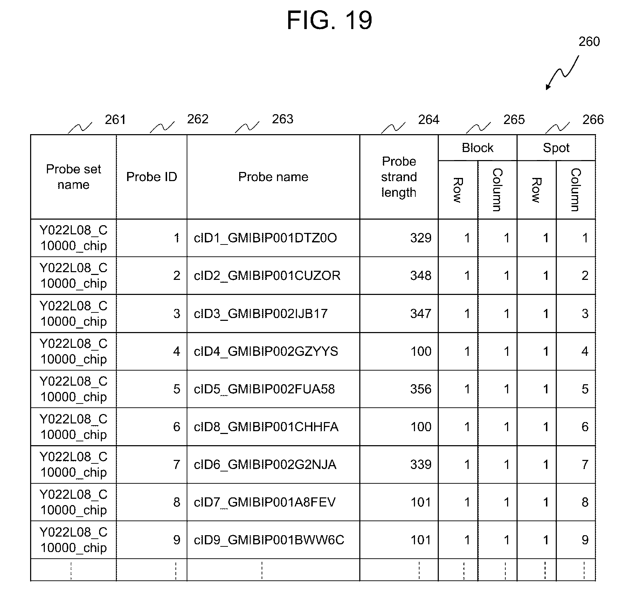

Next, all the representative base sequences of the above cluster set "Y022L08_C10000" were registered in a digital DNA chip file with the name "Y022L08_C10000_chip" as the probe base sequence set for virtual hybridization, and a two-dimensional virtual probe arrangement was determined. The resultant probe base sequence virtual arrangement list 260 is shown in FIG. 19. The probe base sequence virtual arrangement list 260 includes substantially the same information as the probe storage unit 132.

The probe base sequence virtual arrangement list 260 shows virtually the position of the probe base sequence of "Y022L08_C10000_chip" on a flat plate DNA chip substrate when virtually arranged in a rectangular shape. In other words, the positions of the 8,224 types of probe base sequence are identified by first dividing into a block of 24 rows and 4 columns, and then positions within a block are divided into 8 rows and 12 columns. In this example, the number of probe base sequences is large, so only a part of the table is shown in FIG. 19.

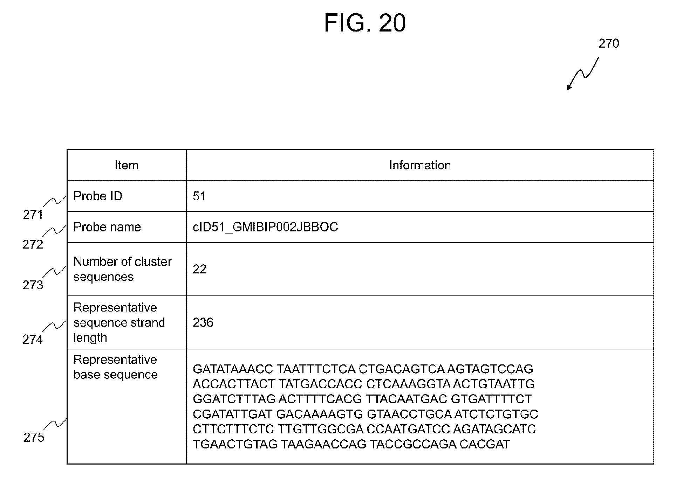

The detailed information of the base sequences of each probe arranged virtually in two-dimensions is shown in the probe detailed information 270 as illustrated in FIG. 20. The detailed information 270 includes probe ID 271 for identifying each probe, probe name 272 which is the name of the probe, the number of cluster sequences 273 which is the number of base sequences of the clusters to which the probe belongs, the representative sequence strand length 274 which is the sequence strand length of the probe, and the representative base sequence 275 which is the base sequence of the probe.

Next, the two files 1.GAC.454Reads.fna and 2.GAC.454Reads.fna were selected from the base sequence data set of the target fragments stored in the nucleic acid information processing device 100, and virtual hybridization of the data set of these two combined and "Y022L08_C10000_chip" was executed with the threshold of the E-value set to 1.0E.

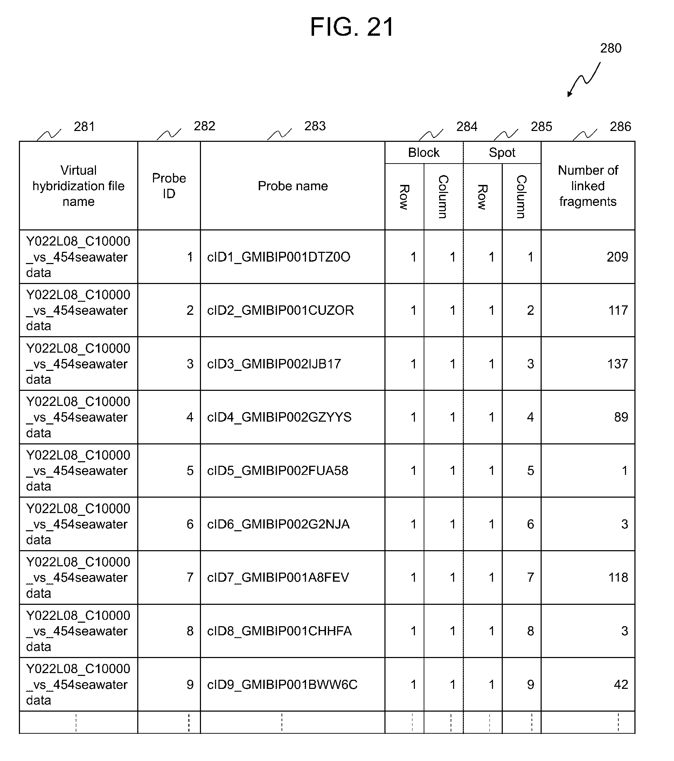

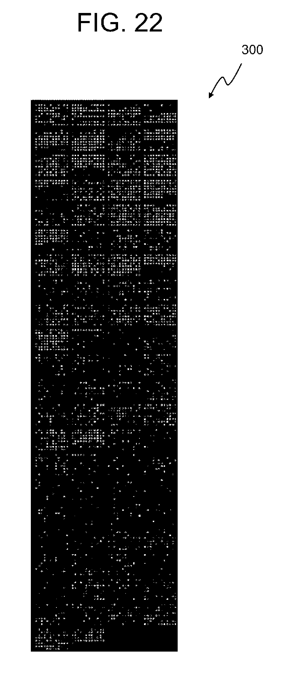

The file of the virtual hybridization results obtained was named "Y022L08_C10000_chip_vs_454 seawater data", which is shown in FIGS. 21 and 22 in two formats. The virtual hybridization results table 280 in FIG. 21 shows "Y022L08_C10000_chip_vs_454 seawater data" as a table of the number of linked fragments for each probe. The virtual hybridization results table 280 includes the virtual hybridization file name 281, the probe ID 282, the probe name 283, the block 284 for identifying the position of the probe on the digital DNA chip, the spot 285 for identifying the position within the block, and the number of linked fragments 286 which is the number of fragments that are similar to the probe. In this example, the number of probe base sequences is large, so only a part of the table is shown.

Also, the image 300 which is a "virtual hybridization image" of FIG. 22 shows a pseudo image of the results in accordance with an image of the DNA microarray. In the image 300, each probe in the probe sequence list "Y022L08_C10000_chip" is shown from upward to downward in FIG. 22 in the order of younger probe base sequence probe ID number. The brighter the color of a spot indicates the greater the number of virtually hybridized target nucleic acid fragments in the probe base sequence arranged virtually at that position. The probe with the greatest number of virtually hybridized target fragments had 10,326 virtually hybridized target nucleic acid fragments.

In this example, the analysis of degree of similarity determined by one to one comparison between the target nucleic acid fragments and the probe base sequences in the virtual hybridization was executed by round robin, and for each probe identified for which the length of the target fragment was greater than or equal to the probe strand length and the base sequence completely matched throughout the whole area of the probe, the probe was counted as a virtual hybridization. Therefore, each of the different parts within the target nucleic acid fragments were counted a plurality of times as virtually hybridized with each different probe.

In this example, using the base sequence data of the microbes in the seawater imported into the nucleic acid information processing device 100, the time required for preparing the probe base sequence list "Y022L08_C10000_chip" by clustering was approximately 30 hours using a grid computer consisting of five computers that incorporated two Xeon X5520 Quad Core 2.26 GHz as CPU and 8-GB RAM, also, the time required for virtual hybridization of "Y022L08_C10000_chip" and a file that linked the two files 1.GAC.454Reads.fna and 2.GAC.454Reads.fna was a total of approximately 30 minutes with the same computer.

In tests using a DNA chip, first the list of the probe base sequences is prepared. Thereafter it is necessary to chemically synthesize all the probe DNA in accordance with the list, determine the positions on a DNA chip substrate or matrix, and fix the probe DNA thereto. Normally, these tasks require several days. In contrast, in the virtual hybridization in this example, by just preparing the probe base sequence list, it is possible to use the data as it is in the virtual hybridization, and the effort and time necessary to prepare the DNA chip is not required. Also, compared with hybridization by testing using a DNA chip which normally requires overnight, the time required for virtual hybridization by information processing using a computer was only about 30 minutes.

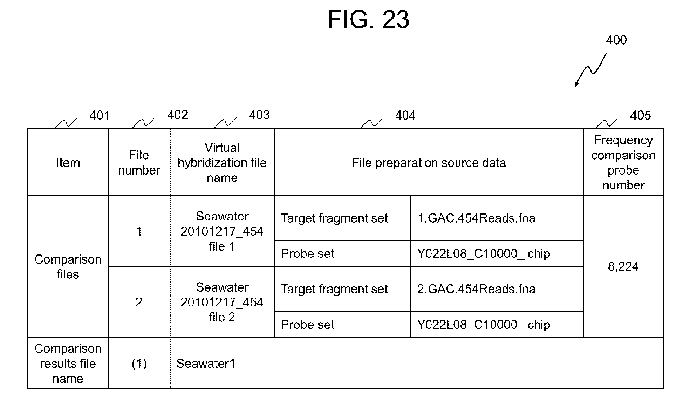

Next, summary table 400 in FIG. 23 shows a comparison of the numbers of target fragments for virtual hybridization with the same probe of the results files seawater 20101217_454 file 1 and seawater 20101217_454 file 2 which were obtained by virtual hybridization of the two target fragment sets 1.GAC.454Reads.fna and 2.GAC.454Reads.fna with the probe set "Y022L08_C10000_chip". Summary table 400 includes items 401, file number 402, Virtual hybridization file name 403, file preparation source data 404, and frequency comparison probe number 405. The time required for this comparison analysis was only about 10 minutes.

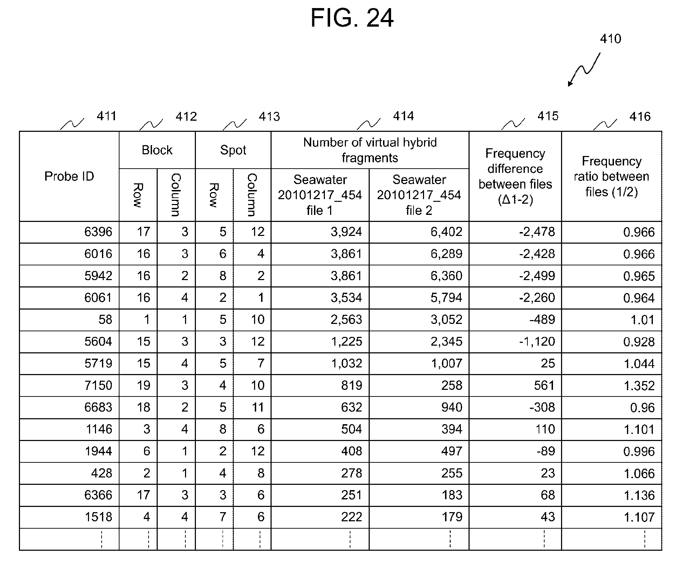

Results display screen 410 showing these results arranged in descending order of probes of virtual hybridization fragments in seawater 20101217_454 file 1 is shown in FIG. 24. The results display screen 410 includes probe ID 411, block 412, spot 413, number of virtual hybridization fragments similar to the probe 414, frequency difference between files 415, and frequency ratio between files 416. Here, the frequency ratio between files 416 is obtained by obtaining the relative values of the number of virtual hybridization fragments 414 for each probe for the two data files seawater 20101217_454 file 1 and seawater 20101217_454 file 2 after normalization and obtaining the ratio between relative values for each probe, in order to correct the data between the two files. In this example, the number of probe base sequences is large, so FIG. 24 shows only a part of the screen. In the results display screen 410, as shown in the second column from the right in FIG. 24 (frequency difference between files 415), the frequency difference between files, which is the difference between the number of virtual hybridization fragments for each probe in the two virtual hybridization results is shown, and as shown in the rightmost column (frequency ratio between files 416), the frequency ratio between files (here, the values in the second decimal place are rounded), which is the ratio of the number of virtual hybridization fragments for each probe in the two virtual hybridization results is shown.

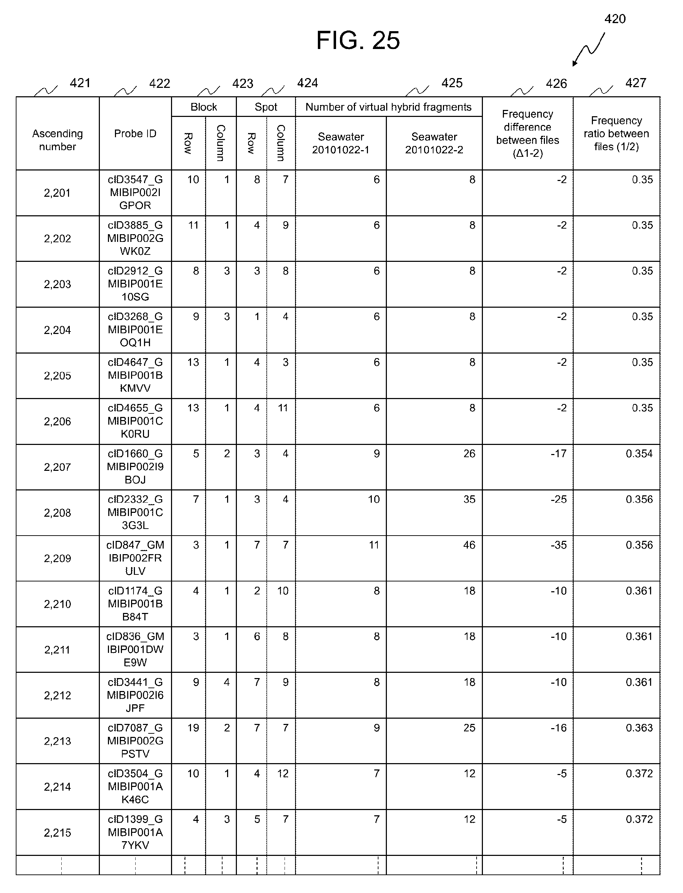

In the results display screen 410, if the data is arranged in order of largest frequency difference, it is possible to detect the probe fragments with a large numerical difference in the two virtual hybridization results. Also, as in the results display screen 420 in FIG. 25, if the data is arranged and displayed in the order of largest frequency ratio between files, it is possible to detect the probe fragments with a large ratio in the two virtual hybridization results. In the results display screen 420, an ascending number 421 for ease of viewing the results is added, and a part in the middle of the whole table is displayed, but otherwise it is basically the same as the results display screen 410 in FIG. 24. In this example, the number of probe base sequences is large, so, in FIG. 25, the results display screen 420 only shows a part in the middle.

As comparison analysis, if, for example, the virtual hybridization results obtained for a seawater target fragment set at point A at a certain time and the virtual hybridization results obtained for a seawater target fragment set at the same point A at a different time are selected, it is possible to extract the base sequences of probe fragments whose quantity or ratio have changed greatly with the passage of time at point A. Also, if target fragments obtained at different points are compared, it is possible to extract the base sequence of probe fragments whose quantity varies greatly with position. If a comparison is executed of the frequency difference or frequency ratio of a number of virtual hybridization fragments between a plurality of target fragments, it is considered that a more accurate comparison can be made if, for example, the numbers are corrected with parameters such as the ratio of quantity of DNA extracted from seawater per unit volume.

As described above, by analyzing base sequence information on a computer with the nucleic acid information processing device 100 using a digital DNA chip prepared in accordance with an embodiment of the present invention, it was possible to execute frequency analysis of similar base sequences while greatly reducing the time and effort.

### REFERENCE NUMERALS

- 1: Imported data
- 2: Processing function
- 3: Database
- 4: Output data
- 100: Nucleic acid information processing device
- 101: Input device
- 102: External memory device
- 103: Calculation device
- 104: Main memory device
- 105: Communication device
- 106: Output device
- 107: Bus
- 110: Control unit
- 130: Storage unit
- 140: Output display unit
- 150: Input receiving unit
- 160: Communication processing unit

## Claims

1. A nucleic acid information processing device, comprising: a storage unit that stores first base sequence information including information on a plurality of base sequences, and second base sequence information including information on a plurality of base sequences;
a threshold value receiving unit adapted to receive information that identifies a similarity threshold;
a hybridization unit adapted to identify a degree of similarity and a starting position and a finishing position of a similar portion for one to one combinations with the base sequences included in the first base sequence information as a target, and the base sequences included in the second base sequence information as a probe; and
a similar base sequence counting unit adapted to count for each probe a number of the targets for which the identified degree of similarity is greater than or equal to the similarity threshold and store the count in the storage unit.

2. The nucleic acid information processing device according to claim 1, wherein
upon a series of base sequences included in the target base sequence corresponding to a series of base sequences included in the probe base sequence, the hybridization unit determines the series of target base sequences to be a similar portion of the probe.

3. The nucleic acid information processing device according to claim 1 or 2, wherein
the similar base sequence counting unit adapted to count for each probe
a) the number of the targets with the degree of similarity greater than or equal to the threshold, for which the similar portion to the base sequence of any probe corresponds to a portion from a starting position to a finishing position of the probe, and
b) the number of sets of linked targets for which the base sequence corresponds to the base sequence of the probe, upon two or more of the target similar portions for which the degree of similarity is greater than or equal to the threshold being linked; and
store the counts in the storage unit.

4. The nucleic acid information processing device according to claim 3, wherein
for the linked targets, the similar base sequence counting unit adapted to identify and link targets satisfying the following conditions:
in the target at a start of the link, a finishing position of the target similar portion is a finishing position of the target,
in the target at an end of the link, a starting position of the target similar portion is a starting position of the target, and
in targets linked between the target at the start of the link and the target at the end of the link, the starting position and the finishing position of the target similar portion are the starting position and the finishing position of the target; and
the linked targets are identified as sets of linked targets.

5. The nucleic acid information processing device according to claim 3, wherein
the similar base sequence counting unit adapted to count for each probe the number of sets of linked targets in which
a plurality of targets for which the starting position is the next position after the finishing position of the similar portion is linked, for the targets for which a similar portion is a portion from a starting position to a finishing position of the base sequence of the target from the targets with a degree of similarity greater than or equal to the threshold,
a target having the similar portion for which the starting position of the similar portion of the target is the starting position of the probe is allocated to be the start of the link, and
a target having a similar portion for which the finishing position of the similar portion of the target is the finishing position of the probe is allocated to be the end of the link.

6. The nucleic acid information processing device according to claim 4 or 5, wherein
the similar base sequence counting unit adapted to link even overlapping similar portions of targets to be linked in the linking process.

7. The nucleic acid information processing device according to any one of claims 1 to 6, further comprising:
a results specification receiving unit adapted to receive specification of two different results information obtained from the results of hybridization of two different first base sequence information with the same second base sequence information; and
an output unit adapted to output the difference of the number of the targets for the same probe included in the specified two results information.

8. The nucleic acid information processing device according to claim 7, wherein
one of the two different first base sequence information is base sequence information obtained from a predetermined object, that is sampled at a different time to the other of the two different first base sequence information.

9. The nucleic acid information processing device according to claim 8, wherein
the predetermined object is a mixture of biological material that includes a plurality of bion, parts, tissues, cells, or a mixture thereof.

10. The nucleic acid information processing device according to claim 8 or 9, wherein
the predetermined object is an object sampled from a predetermined geographical position.

11. A method of processing nucleic acid information with a nucleic acid information processing device,
the nucleic acid information processing device comprising:
a storage unit that stores first base sequence information that includes information of a plurality of base sequences, and second base sequence information that includes information of a plurality of base sequences, and a processing unit;
the processing unit executing:
a threshold value receiving step of receiving information for identifying a similarity threshold;
a hybridization step of identifying a degree of similarity and a starting position and a finishing position of a similar portion for one to one combinations with the base sequences included in the first base sequence information as a target, and the base sequences included in the second base sequence information as a probe; and
a similar base sequence counting step of counting for each probe a number of the targets for which the identified degree of similarity is greater than or equal to the similarity threshold, and storing the count in the storage unit.

12. The method of processing nucleic acid information according to claim 11, wherein
in the hybridization step, upon a series of base sequences included in the target base sequence corresponding to a series of base sequences included in the probe base sequence, the series of the target base sequences is determined to be a similar portion of the probe.

13. The method of processing nucleic acid information according to claim 11 or 12, wherein
in the similar base sequence counting step,
a) the number of the targets with a degree of similarity greater than or equal to the threshold, for which the similar portion to the base sequence of any probe corresponds to the portion from the starting position to the finishing position of the probe, and
b) the number of sets of linked targets for which the base sequence corresponds to the base sequence of the probe, when two or more of the target similar portions for which the degree of similarity is greater than or equal to the threshold are linked
are counted for each probe and the counts are stored in the storage unit.

14. The method of processing nucleic acid information according to claim 13, wherein
in the similar base sequence counting step, for the linked targets, targets satisfying the following conditions:
in the target at the start of the link, the finishing position of the similar portion of the target is the finishing position of the target,
in the target at the end of the link, the starting position of the similar portion of the target is the starting position of the target, and
in targets linked between the target at the start of the link and the target at the end of the link, the starting position and the finishing position of the similar portion of the target are the starting position and the finishing position of the target
are identified and linked, and the linked targets are identified as sets of linked targets.

15. The method of processing nucleic acid information according to claim 13, wherein
in the similar base sequence counting step, the number of sets of linked targets is counted for each probe in which
a plurality of targets for which the starting position is the next position after the finishing position of the similar portion is linked, for the targets for which the similar portion is a portion from the starting position to the finishing position of the base sequence of the target from the targets with the degree of similarity greater than or equal to the threshold,
a target having a similar portion for which the starting position of the similar portion of the target is the starting position of the probe is allocated to be the start of the link, and
a target having a similar portion for which the finishing position of the similar portion of the target is the finishing position of the probe is allocated to be the end of the link.

16. The method of processing nucleic acid information according to claim 14 or 15, wherein
in the similar base sequence counting step,
even overlapping similar portions of targets to be linked are linked in the linking process.

17. The method of processing nucleic acid information according to any one of claims 11 to 16, further comprising:
a results specification receiving step of receiving specification of two different results information obtained from the results of hybridization of two different first base sequence information with the same second base sequence information; and
an output step of outputting the difference of the number of the targets for the same probe included in the specified two results information.

18. The method of processing nucleic acid information according to claim 17, wherein
one of the two different first base sequence information is base sequence information obtained from a predetermined object, that is sampled at a different time to the other of the two different first base sequence information.

19. The method of processing nucleic acid information according to claim 18, wherein
the predetermined object is a mixture of biological material that includes a plurality of bion, parts, tissues, cells, or a mixture thereof.

20. The method of processing nucleic acid information according to claim 18 or 19, wherein
the predetermined object is an object sampled from a predetermined geographical position.
